# EUROPEAN PATENT APPLICATION

(11) **EP 4 692 314 A1**
(43) Date of publication of application: **11.02.2026**
(21) Application number: 24777912.7
(22) Date of filing: 22.03.2024
(51) Int. Cl.: C12N 1/21, C12N 1/15, C12N 1/19, C12N 15/70

(54) **HOST CELL FOR IMPROVING STABILITY OF PLASMID**

(30) Priority: 24.03.2023 CN 202310316764
(71) Applicant: Nanjing GenScript Biotech Co., Ltd., Nanjing, Jiangsu 211100 (CN)
(72) Inventor: MENG, Qingwei, Nanjing, Jiangsu 211100 (CN); ZHANG, Kun, Nanjing, Jiangsu 211100 (CN); ZHU, Xiuxiu, Nanjing, Jiangsu 211100 (CN); HU, Ting, Nanjing, Jiangsu 211100 (CN)
(74) Representative: Ström & Gulliksson AB
(86) International application number: PCT/CN2024/083274
(87) International publication number: WO 2024/199123

(57) **Abstract**

Provided is a host cell for improving the stability of a plasmid. By means of knocking out sbcC and/or sbcD genes in a cell and knocking out one or more genes of nfi, mutL, ruvA, ruvB and ruvC, or knocking in lacIq or lacIql genes, the stability of the plasmid, which has a hairpin structure sequence, is improved.

## Description

### Cross Reference to Related Applications

This application claims the priority of Chinese Patent Application No. 202310316764.0 filed on March 24, 2023, which is incorporated herein by reference in its entirety.

### Technical Field

The present invention relates to an engineered *E. coli* strain for improving the structural stability of a DNA hairpin, such as an inverted terminal repeat (ITR), particularly to a method and host cell for increasing the structural stability of a wild-type or modified ITR derived from the adeno-associated virus (AAV) genome.

### Background Art

Adeno-associated virus (AAV) is a small, non-pathogenic virus of the Parvoviridae family. Unlike other members of the family, AAV relies on helper viruses for replication. The AAV genome is about 5 kb, and consists of a single-stranded DNA of positive or negative polarity. The ends of the genome are short palindromes/inverted terminal repeats (ITRs) that can fold into a hairpin structure which serves as the origin of replication of viral DNA. Recombinant adeno-associated virus (rAAV), as a DNA delivery vector, has become the most important vector in gene research and gene therapy due to its advantages of high safety, low immunogenicity, broad applicability, long duration of action, etc.

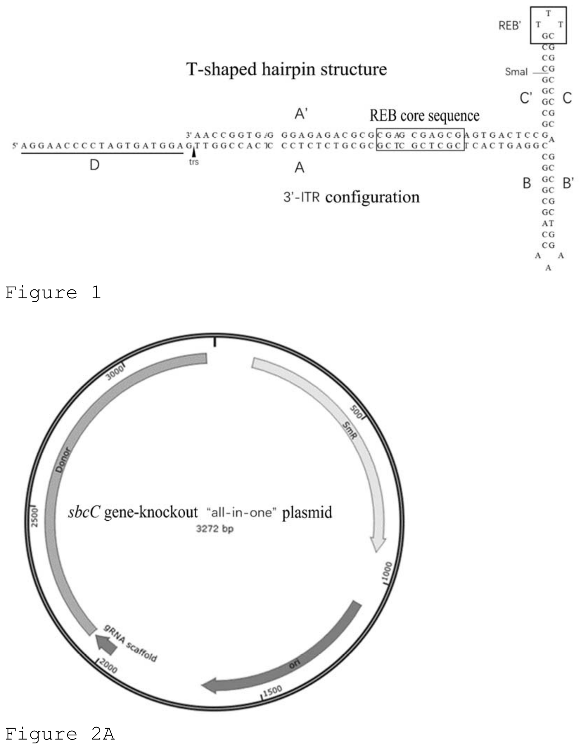

To date, many different serotypes of AAV have been isolated from humans or primates (Govindasamy et al., "Structurally Mapping the Diverse Phenotype of Adeno-Associated Virus Serotype 4, " J. Vir., 80 (23): 11556-11570 (2006)). For example, the AAV2 genome has a length of 4680 nucleotides, and contains two open reading frames. The ends of its genome are short 145-bp ITRs, which are the sole cis-acting elements in the rAAV genome and are involved in integration, rescue, replication, and packaging of the genome. The outer 125 nt (5' A-B-B'-C-C'-A' 3' or 5' A-C-C'-B-B'-A' 3') of the ITRs form a unique self-complementary T-shaped palindromic hairpin structure (FIG. 1), with the sequence as set forth in SEQ ID NO: 10. This palindromic structure consists of one longer palindromic arm (A-A' region) and two shorter palindromic arms (B-B' and C-C' regions). The remaining 20 nt are D regions, which have a single-stranded structure.

The palindromes of AAV-ITRs form a highly stable T-shaped hairpin structure with high GC content, making them prone to be lost due to mutations or recombinations during bacterial replication. Jude Samulski, a pioneer in the AAV gene therapy field, emphasized the importance of ITR integrity for efficient replication and packaging of rAAV in a review, and suggested that ITR deletions should preferably be less than 20% (Flotte, T. R., and B. J. Carter. "Adeno-associated virus vectors for gene therapy." Gene therapy 2.6 (1995): 357-362. Current protocols in human genetics 23.1 (1999): 12-9.). The Chinese regulations emphasize that "the integrity and homogeneity of recombinant vector genomes or plasmids shall be tested" ("General Chapter for Human Gene Therapy Products", Chinese Pharmacopoeia 2020 Edition). The FDA regulations emphasize the necessity to "Confirm the vector is stable during manufacturing" and to "Demonstrate similar purity between lots used in IND-enabling preclinical studies and clinical lots" (Chemistry, Manufacturing, and Control (CMC) Information for Human Gene Therapy Investigational New Drug Applications (INDs)). Single-molecule real-time sequencing enables comprehensive analysis of the rAAV genome as a single intact molecule. Additional studies have found that in the HEK293 triple-plasmid packaging system, deleted ITRs are associated with the packaging of non-vector sequences, and sequencing results have shown that rAAV contains 1.3% to 2.3% of these undesirable chimeras, which are composed of host cell genomic sequences and ITR sequences (Tai, Phillip WL, et al. "Adeno-associated virus genome population sequencing achieves full vector genome resolution and reveals human-vector chimeras." Molecular Therapy-Methods & Clinical Development 9 (2018): 130-141.). The effects of ITR deletions on rAAV viral packaging and its application in gene therapy are complex, as vector-host interactions may give rise to unexpected biological consequences. Using incomplete ITR sequences in the preclinical research stage will reduce the reproducibility of experiments within and across laboratories, and may affect the reproducibility of experiments of clinical trials using complete ITR sequences, delaying their translation into clinical applications. Since ITRs have the capacity for self-repair during vector packaging in some cases, partial deletions of ITRs may go unnoticed, a fact that further exacerbates this issue (Wilmott, P., Lisowski, L., Alexander, I. E., & Logan, G. J. (2019). A user's guide to the inverted terminal repeats of adeno-associated virus. Human gene therapy methods, 30(6), 206-213.).

There is currently no good way to avoid ITR deletions during rAAV-ITR plasmid construction and *E. coli* amplification. Some recombination-deficient commercial strains such as JC8111, SURE2, Stabl3, and XL10-Gold are often used for the construction and amplification of plasmids containing ITR structures, but they are not always effective. Therefore, there is a need to develop high-yield engineered *E. coli* production strains for manufacturing vectors containing palindromes and inverted terminal repeats. The sbcC-sbcD nuclease, a member of the structural maintenance of chromosomes (SMC) protein family, can cleave hairpin DNA (Connelly, John C., Lucy A. Kirkham, and David RF Leach. "The ΔsbcC-sbcD nuclease of Escherichia coli is a structural maintenance of chromosomes (SMC) family protein that cleaves hairpin DNA." Proceedings of the National Academy of Sciences 95.14 (1998): 7969-7974.). *E. coli* sbcC mutants allow the stable propagation of DNA replicons containing long palindromes (Chalker, Alison F., David RF Leach, and Robert G. Lloyd. "Escherichia coli sbcC mutants permit stable propagation of DNA replicons containing a long palindrome." Gene 71.1 (1988): 201-205.). Palindromes such as shRNAs or AAV ITRs are more stable in sbcC-knockout strains than in non-knockout strains. GT115 (F- mcrA Δ(mrr-hsdRMS-mcrBC) φ80lacZΔM15 ΔlacX74 nupG recA1 araD139 Δ(ara-leu)7697 galE15 galK16 rpsL(StrA) endA1 Δdcm uidA(ΔMluI)::pir-116 ΔsbcC-sbcD.) contains sbcC and sbcD gene knockouts. This modification significantly increases the number of recombinant clones containing plasmids with intact hairpin structures. However, it is still impossible to achieve a sufficiently high ITR integrity, particularly when the length of the inverted terminal repeats in the A-A' region exceeds 30 bp. Even in the ΔsbcC-sbcD mutant strain, there are obvious deletions.

The lac operon and its control are fundamental concepts in transcriptional regulation introduced in introductory and many advanced molecular biology courses. The promoter gene (lacP) of the lac operon is the binding site for RNA polymerase during transcription initiation. A fundamental principle regarding lacP is that it is "leaky", meaning that the transcriptional control of the operon is not 100% efficient, and in wild-type cells, transcription from the promoter will never be completely "turned off" and there is always some basal transcription (F. Jacob, J. Monod (1961) Genetic regulatory mechanisms in the synthesis of proteins, J. Mol. Biol. 3, 318-356.). Due to its well-characterized nature, lacP is often used in plasmid cloning vectors and for expressing recombinant proteins in bacteria. This promoter provides the ability to allow high-level expression of the introduced gene. For expression cloning, structures are usually constructed in medium- and high-copy-number plasmids (such as pUC, pBluescript, and pET vectors and their derivatives) to increase the copy number of recombinant genes. However, due to the high copy number of genes, promoters, and operator sites, this may increase leaky expression. (Nielsen, Brent L., Van C. Willis, and Chin-Yo Lin. "Western blot analysis to illustrate relative control levels of the lac and ara promoters in Escherichia coli." Biochemistry and Molecular Biology Education 35.2 (2007): 133-137.). During gene transcription, RNA polymerase first binds to the promoter position upstream of the transcription start site on the DNA template. The breaking of hydrogen bonds in the local DNA double helix structure at the binding site (and the unwinding of the local DNA double helix structure) leads to the formation of excessive super coils near the locally unwound region, which, in turn, causes the deletion of hairpin structures. We hypothesize that frequent unwinding contributes to the deletion of hairpin structures, especially in the AAV-ITR structures.

### Summary of the Invention

In one aspect, provided herein is a host cell, wherein the genome of the host cell has sbcC and/or sbcD genes knocked out, and the host cell further comprises the following modifications:
1) one or more of the nfi, mutS, mutL, mutH, ruvA, ruvB, and ruvC genes are knocked out in the genome of the host cell; and/or
2) the lacIq or lacIq1 gene is knocked into the genome of the host cell.

In some embodiments, in the genome of the host cell, the sbcC or sbcD gene is knocked out.

In some embodiments, in the genome of the host cell, the sbcC and sbcD genes are knocked out.

In some embodiments, in the genome of the host cell:
1) the sbcC and/or sbcD genes are knocked out, and the nfi gene is knocked out;
2) the sbcC and/or sbcD genes are knocked out, and at least one of the ruvA, ruvB, and ruvC genes is knocked out;
3) the sbcC and/or sbcD genes are knocked out, and at least one of the mutS, mutL, and mutH genes is knocked out; or
4) the sbcC and/or sbcD genes are knocked out, and the lacIq or lacIq1 gene is knocked in.

In some embodiments, in the genome of the host cell:
1) the sbcC and sbcD genes are knocked out, and the nfi gene is knocked out;
2) the sbcC and/or sbcD genes are knocked out, and the ruvA and ruvB genes are knocked out;
3) the sbcC and/or sbcD genes are knocked out, and the ruvC gene is knocked out;
4) the sbcC and/or sbcD genes are knocked out, and the ruvA, ruvB, and ruvC genes are knocked out;
5) the sbcC and/or sbcD genes are knocked out, and the mutL gene is knocked out;
6) the sbcC and/or sbcD genes are knocked out, and the mutL and nfi genes are knocked out; or
7) the sbcC and/or sbcD genes are knocked out, the mutL gene is knocked out, and the lacIq or lacIq1 gene is knocked in.

In some embodiments, in the genome of the host cell:
1) the sbcC and sbcD genes are knocked out, and the ruvA, ruvB, and ruvC genes are knocked out; or
2) the sbcC and sbcD genes are knocked out, and the ruvC gene is knocked out.

In some embodiments, the lacIq gene comprises the nucleotide sequence set forth in SEQ ID NO: 8, or a nucleotide sequence having at least 90% sequence identity to the nucleotide sequence set forth in SEQ ID NO: 8; and the lacIq1 gene comprises the nucleotide sequence set forth in SEQ ID NO: 9, or a nucleotide sequence having at least 90% sequence identity to the nucleotide sequence set forth in SEQ ID NO: 9.

In some embodiments, the host cell is an *E. coli* cell.

In some embodiments, the *E*. *coli* cell is a Top10, JM108, or NEB Stable cell.

In another aspect, provided herein is use of the above host cell in the preparation of a nucleic acid molecule comprising a hairpin structure. In particular, provided herein is use of the above host cell in the replication or expression of a nucleic acid molecule comprising a hairpin structure.

In some embodiments, the nucleic acid molecule can be a DNA sequence or an RNA sequence. In other embodiments, the nucleic acid molecule is a plasmid.

In some embodiments, the hairpin structure is an ITR sequence, preferably an ITR derived from an adeno-associated virus.

In some embodiments, the number of nucleotides in the A region in the ITR sequence is at least 20; preferably at least 30; and more preferably at least 40.

In some embodiments, the host cell is an *E. coli* cell; preferably a Top10, JM108, or NEB Stable cell.

In some embodiments, the hairpin structure sequence has stability in the host cell, including replication stability and/or passage stability.

### Brief Description of the Drawings

FIG. 1 shows the schematic diagram of the AAV2-ITR hairpin structure.

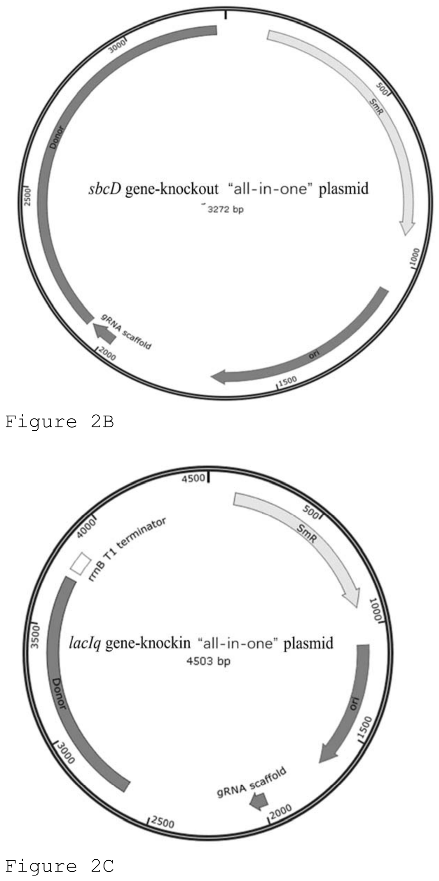

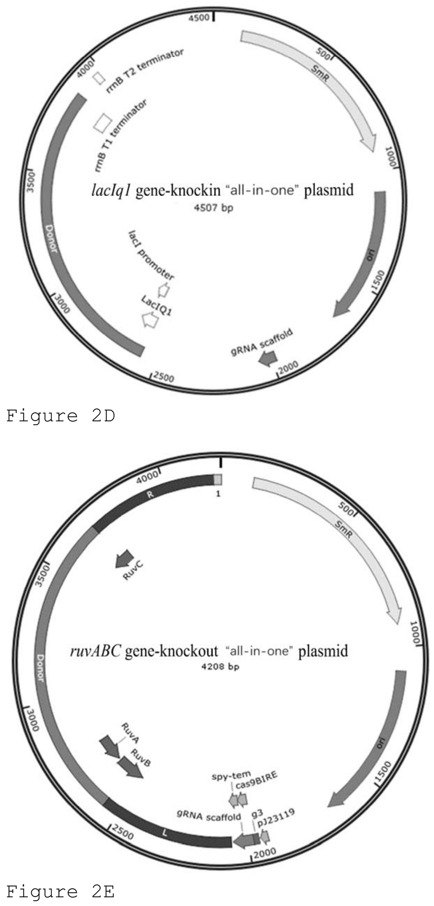

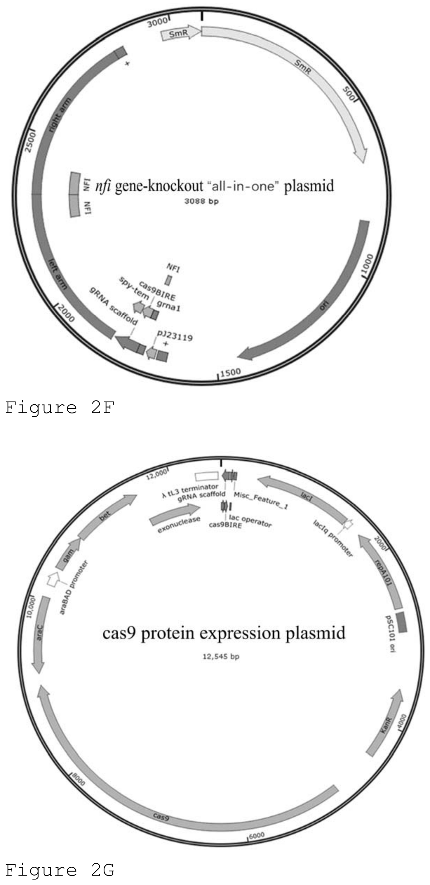

FIGs. 2A-2G show the schematic diagrams of the plasmid maps of the "all-in-one" plasmids used for gene knockout or knockin and the Cas9 protein expression plasmid in some examples. FIG. 2A shows the plasmid map of the sbcC gene-knockout plasmid; FIG. 2B shows the plasmid map of the sbcD gene-knockout plasmid; FIG. 2C shows the plasmid map of the lacIq gene-knockin plasmid; FIG. 2D shows the plasmid map of the lacIq1 gene-knockin plasmid; FIG. 2E shows the plasmid map of the ruvABC gene-knockout plasmid; FIG. 2F shows the plasmid map of the nfi gene-knockout plasmid; and FIG. 2G shows the plasmid map of the Cas9 protein expression plasmid.
FIGs. 3A-3C show the schematic diagrams of the plasmid maps of the test plasmids in some examples. FIG. 3A shows the schematic diagram of the pITR-test1 plasmid; FIG. 3B shows the schematic diagram of the pITR-test2 plasmid; and FIG. 3C shows the schematic diagram of the pITR-test3 plasmid.
FIGs. 4A-4C show the Sanger sequencing chromatograms of the plasmids with intact ITRs (FIG. 4A), impure ITRs (FIG. 4B), and deleted ITRs (FIG. 4C) in some examples.
FIG. 5 shows the SmaI restriction digestion results of pITR-test1 in the fourth generation of different strains in the examples. Lanes 1, 3, 5, 7, and 9 show the gel images of the extracted plasmids, and lanes 2, 4, 6, 8, and 10 show the corresponding gel images after Smal restriction digestion. The theoretical band sizes after Smal restriction digestion are 2646 bp, 1681 bp, and 1544 bp.
FIG. 6 shows the fermentation curve of the AAV plasmid fermented by the JM108[ΔsbcC-sbcDΔmutL lacIq] strain in the examples.
FIG. 7 shows the Sanger sequencing results of the ITR regions of the AAV plasmid fermented by the JM108[ΔsbcC-sbcDΔmutL lacIq] strain in the examples.
FIG. 8 shows the average supercoil ratio results of strains with different recombinant genotypes in the examples.

### Detailed Description of Embodiments

Unless otherwise stated, all technical and scientific terms used herein have the meanings commonly understood by those of ordinary skill in the art.

The term "or" refers to an individual element of the listed optional elements, unless the context explicitly indicates otherwise. The term "and/or" refers to any one of, any two of, any three or more of, or all of the listed optional elements.

The term "comprising" or "including" refers to the inclusion of the stated elements, integers, or steps, but not the exclusion of any other elements, integers, or steps. When the term "comprising" or "including" is used herein, unless otherwise indicated, the situations where the subject matter consists of the described elements, integers, or steps are also encompassed.

The term "recombinant adeno-associated virus vector" refers to a recombinant, non-replicating adeno-associated virus. A recombinant adeno-associated virus (rAAV) vector comprises a protein capsid of a specific serotype encapsidating a recombinant genome, which comprises functional 5' and 3' inverted terminal repeats (ITRs), between which an exogenous gene expression cassette that replaces the rep gene expression cassette and/or the cap gene expression cassette of the wild-type AAV is linked. The ITR sequences provide functional rescue, replication, and packaging for rAAV. In some examples, the ITR sequences are derived from AAV2. The exogenous gene expression cassette usually consists of a series of expression regulatory elements and a coding region.

The term "hairpin structure" herein refers to a nucleotide sequence that has a circular structure, a base-pairing structure, and the like. The hairpin structure can be a nucleotide sequence that contains palindromes or a nucleotide sequence that contains structures such as a Holliday junction. Due to its complex structure, the hairpin structure is difficult to replicate in the cell and is poorly stable. In particular, exogenous hairpin structure genes are susceptible to cleavage by various nucleases in the host cell, making it difficult to form complete sequences.

The term "inverted terminal repeat (ITR)" refers to the regions recognized in the art found at the 5' and 3' ends of the AAV genome that function in *cis* as a DNA origin of replication and packaging signal in the viral genome. The AAV ITRs, together with the AAV rep-coding region, provide efficient excision and rescue, as well as the integration of the nucleotide sequence inserted between the two flanking ITRs into the host cell genome. Yan et al., disclosed the sequences of some AAV-related ITRs (Yan et al., J. Virol. 79(1): 364-379 (2005)). The ITR sequence that finds use herein can be a full-length wild-type AAV ITR or a functional fragment thereof, or a full-length sequence variant of a wild-type AAV ITR that can function in *cis* as an origin of replication. The AAV ITRs useful in the mutant host of the present invention may be derived from any known AAV serotype, and in certain preferred embodiments, from the AAV2 serotype.

The terms *"Escherichia coli"* and *"E. coli"* are used interchangeably. It should be understood that the terms include, but not be limited to, *E. coli* deposited cultures from the Department of Bacteriology at Stanford University, and all derivatives of Lederberg strain W1485, which are generated from the original *E. coli* K-12 strain following treatment with UV light, X-rays, and/or other chemical or genetic treatments (Bachmann, B. J. 1987. Derivations and genotypes of some mutant derivatives of Escherichia coli K-12, p. 1191-1219. J. L. 1ngraham, K. B. Low, B. Magasanik, M. Schaechter, and H. E. Umbarger (eds.), Escherichia coli and Salmonella typhinurium: cellular and molecular biology. American Society for Microbiology, Washington, D.C.); and clonal offsprings of a *Bacillus coli* strain (strain B) from the Pasteur Institute (Luria, SE & Anderson, TF, 1942, Proc. Natl. Acad. Sci. U.S.A. 28 127-130; and Daegelen, P et al., 2009, J. Mol. Biol. 394 634-43-NCBI Taxonomy ID 37762); preferably cells derived from the K-12 strain, more preferably recA endA gene-knockout cells, such as Top10, DH5alpha, and JM108 which are preferred in some embodiments. MutL is one of the proteins involved in mismatch repair. The term "mismatch repair (MMR)" herein refers to the process by which a host cell uses a protein (or enzyme) to remove unpaired base sites in a DNA molecule. Such mismatches are usually caused by misincorporation of bases during DNA replication. Inactivation or deletion of these proteins (or enzymes) associated with mismatch repair may result in an increase in the base mutation rate, the occurrence of frameshift mutations and/or abnormal recombination between homologous sequences in DNA molecules. In *E. coli,* the proteins involved in mismatch repair mainly include MutS, MutL, MutH, and UvrD (DNA helicase II), whose deletions result in *E. coli* exhibiting a defect in the methylation-mediated mismatch repair system. In addition, the Dam methylase can mark which strand is used as the parent strand and which strand as the daughter strand in mismatch repair. The Dam methylase recognizes the adenine residue on GATC in the DNA sequence and methylates it at the N-6 position. During DNA replication, the GATC in the nascent daughter strand is not methylated by the Dam methylase in time, allowing the strand to be recognized as the daughter strand. The mismatch repair process is generally as follows. The MutS dimer recognizes and binds to the DNA mismatched base site, and the MutL dimer binds to the MutS dimer to form a tetrameric complex, which can activate the endonuclease activity of MutH, cleaving the unmethylated DNA strand (daughter strand) at the GATC sequence near the mismatch site, thereby generating a single-stranded nick; in which the MutL protein may play an important role in recruiting the DNA polymerase III subunit to participate in the post-excision resynthesis during mismatch repair. UvrD initiates unwinding from the single-stranded nick in the direction toward the mismatch, and subsequently, with the assistance of an exonuclease (e.g., Exo I and Exo VII), degrades the unwound single strand. The single-stranded binding protein (SSB) binds to and stabilizes the template strand (parent strand) until DNA polymerase and ligase fill in the excised region based on the sequence of the template strand, thereby completing the entire repair process.

Holliday junction is a key intermediate in the recombination repair process. In the classical RecA-mediated homologous recombination repair process, the RecA-ssDNA-dsDNA complex forms a Holliday junction structure, which can undergo strand displacement via RuvABC. Studies have shown that in addition to homologous recombination, many hairpin DNAs form Holliday junction structures, such as a hairpin or cruciform formed by reverse complementarity, an intermolecular Holliday junction formed by base-complementary pairing of free DNA single strands of H DNA, a triple-stranded junction structure formed by strand slippage during the replication of tandem repeated DNAs, and replication fork reversal caused by blocked DNA replication. These structures may all be the target of RuvABC. Because these structures are usually present in the hairpin DNAs, RuvABC causes the hairpin sequences to be unstable. RuvABC is a complex consisting of three proteins-RuvA, RuvB, and RuvC, and can mediate branch migration and resolve Holliday junctions generated during bacterial homologous recombination. RuvA and RuvB bind to the four-stranded DNA structure formed in the Holliday junction intermediate, and the complex is referred to as the RuvAB complex. The RuvAB complex may exhibit DNA helicase activity, which facilitates the unwinding of a double-stranded DNA. Binding of the RuvC protein to the RuvAB complex is thought to induce the cleavage of the DNA strands, thereby resolving the Holliday junction. Herein, ΔruvABC refers to simultaneous knockout of the ruvA, ruvB, and ruvC genes; and ΔruvAB refers to simultaneous knockout of the ruvA and ruvB genes.

"SbcC" and "SbcD" form a complex in *E. coli,* which plays an important role in DNA repair and genomic stability. The SbcC-SbcD protein complex exhibits nuclease activity and can generate a double-stranded nick in a DNA molecule containing palindromes, thereby leading to its degradation or the generation of mutations (e.g., deletions). It is reported in the literature that mutations or deletions in the sbcC or sbcD gene in *E. coli* can enable a DNA molecule containing palindromes to replicate stably therein.

The nfi gene encodes the DNA repair enzyme endonuclease V (EndoV), which recognizes and cleaves DNA at deaminated adenine lesions (hypoxanthine). In addition, EndoV cleaves DNAs containing various helical distortions, such as loops, hairpins, and flap DNAs. The ends of the B-B' and C-C' arms of the ITR hairpin structure contain loop regions consisting of AAA and TTT. Nanopore data indicate that these sites are prone to deletions, and the resulting REB' plays an important role in the function of the ITRs. Therefore, we hypothesize that knockout of nfi can improve the stability of ITR structures.

lacI is the regulatory gene of the lac operon in E. coli, and the repressor protein it expresses serves as an inhibitor of the lac operator. This repressor protein can bind to an excess of lactose, leading to the loss of its inhibitory effect on the operator and allowing the structural genes of the lac operon-lacZ (β-galactosidase), lacY (permease), and lacA (acetyltransferase)-to be expressed normally. IPTG (isopropyl β-D-1-thiogalactopyranoside), as an analog of lactose, binds to the LacI repressor protein, preventing the operator from being inhibited. Consequently, IPTG is often used as an inducer of the lac operon. The lacIq genotype results from a variation in the lacI gene that causes it to express a quantity of the repressor protein, thereby leading to nearly complete inhibition of the lac operator. When using strains with this genotype for gene expression, the expression of the gene of interest can be more effectively controlled manually. lacIq1 exhibits stronger activity than the lacIq promoter in controlling repressor synthesis.

The term "knockout" herein refers to reducing and ultimately eliminating the expression products of specific genes or their activity. A variety of methods are known in the art for knockout operations, such as by gene editing, e.g., CRISPR gene editing system technology. Those skilled in the art will understand that in most cases, gene knockout does not mean a complete disappearance of the activity of the gene or its expression product. Therefore, after genetic manipulation, if the activity of a gene or its expression product is less than 50%, 40%, 30%, 20%, or less of the original activity, the gene can be considered knocked out. Herein, knockout methods include, but are not limited to, inducing mutations, insertions, or deletions of nucleotides in the gene.

The term "knockin" refers to the addition of a DNA sequence or its fragment into the genome. Such DNA sequences to be knocked in may comprise one or more complete genes, and regulatory sequences associated with the genes or any portion or fragment of the aforementioned genes. For example, a cDNA encoding a wild-type protein can be inserted into the genome of a cell carrying a mutant gene. Knockin strategies do not require the complete or partial replacement of defective genes. In some cases, a knockin strategy may further involve replacing an existing sequence with a provided sequence, for example replacing a mutant allele with a wild-type copy.

The terms "host cell", "strain", and "cell" herein all refer to any cell into which an exogenous nucleic acid molecule (such as an expression vector) may be introduced and in which the exogenous nucleic acid molecule is capable of replicating or being expressed. In some embodiments, the host cell is a prokaryotic cell, such as a Gram-negative bacterium, such as *E. coli.* In some embodiments, the selected host cell is of the JM108, Top10, or NEB Stable strain.

The term "stability" herein refers to the stability of the hairpin structure expressed in the host cell, including the integrity and purity of the hairpin structure gene, etc. Herein, the term "integrity" refers to the consistency between the ITR sequence expressed by the strain and the ITR sequence of interest introduced into the strain; and the term "integrity ratio" refers to the proportion of strains expressing ITR with 100% integrity among all strains expressing ITR. In some embodiments, the hairpin structure gene expressed by the host cell is an ITR gene. In some examples, the integrity ratio of the hairpin structure gene or ITR gene expressed by the host cell of the present invention reaches 50%, 60%, 70%, or 80% or higher; and in other embodiments, the purity of the hairpin structure gene or ITR gene expressed by the host cell of the present invention reaches 50%, 60%, 70%, or 80% or higher. In some embodiments, the stability includes the ability of an exogenously expressed gene to maintain structural integrity after replication in the host cell. In some examples, after four generations of replication, the integrity ratio of the hairpin structure gene or ITR gene expressed by the host cell reaches 50%, 60%, 70%, or 80% or higher; and in other embodiments, after four generations of replication, the purity of the hairpin structure gene or ITR gene expressed by the host cell reaches 50%, 60%, 70%, or 80% or higher.

Provided herein are some host cells, in which the sbcC and/or sbcD genes are knocked out, and additionally, one or more of the nfi, mutL, ruvA, ruvB, and ruvC genes in the cells are knocked out.

In some examples, the sbcC, sbcD, and nfi genes in the cells are knocked out. In other examples, the sbcC, sbcD, and ruvA genes in the cells are knocked out. In other examples, the sbcC, sbcD, and ruvB genes in the cells are knocked out. In other examples, the sbcC, sbcD, and ruvC genes in the cells are knocked out. In other examples, the sbcC, sbcD, and mutL genes in the cells are knocked out. In other examples, the sbcC, sbcD, ruvA, and ruvB genes in the cells are knocked out. In other examples, the sbcC, sbcD, ruvA, ruvB, and ruvC genes in the cells are knocked out. In other examples, the sbcC, ruvA, and ruvB genes in the cells are knocked out. In other examples, the sbcC and ruvC genes in the cells are knocked out. In other examples, the sbcC, ruvA, ruvB, and ruvC genes in the cells are knocked out. In other examples, the sbcD, ruvA, and ruvB genes in the cells are knocked out. In other examples, the sbcD and ruvC genes in the cells are knocked out. In other examples, the sbcD, ruvA, ruvB, and ruvC genes in the cells are knocked out. In other examples, the sbcC and mutL genes in the cells are knocked out. In other examples, the sbcD and mutL genes in the cells are knocked out. In other examples, the sbcC and nfi genes in the cells are knocked out. In other examples, the sbcD and nfi genes in the cells are knocked out. In other examples, the sbcC, sbcD, mutL, and nfi genes in the cells are knocked out. In other examples, the sbcC, mutL, and nfi genes in the cells are knocked out. In other examples, the sbcD, mutL, and nfi genes in the cells are knocked out. In some examples, the mutS, mutH, or UvrD gene in the cells is knocked out instead of knocking out the mutL gene.

In some examples, the sbcC and/or sbcD genes in the cells are knocked out, and further, the ruvA, ruvB, and mutL genes are knocked out. In some examples, the sbcC and/or sbcD genes in the cells are knocked out, and further, the ruvA, ruvB, and nfi genes are knocked out. In some examples, the sbcC and/or sbcD genes in the cells are knocked out, and further, the ruvA, ruvB, mutL, and nfi genes are knocked out. In some examples, the sbcC and/or sbcD genes in the cells are knocked out, and further, the ruvC and mutL genes are knocked out. In some examples, the sbcC and/or sbcD genes in the cells are knocked out, and further, the ruvC and nfi genes are knocked out. In some examples, the sbcC and/or sbcD genes in the cells are knocked out, and further, the ruvC, mutL, and nfi genes are knocked out. In some examples, the sbcC and/or sbcD genes in the cells are knocked out, and further, the ruvA, ruvB, ruvC, and mutL genes are knocked out. In some examples, the sbcC and/or sbcD genes in the cells are knocked out, and further, the ruvA, ruvB, ruvC, and nfi genes are knocked out. In some examples, the sbcC and/or sbcD genes in the cells are knocked out, and further, the ruvA, ruvB, ruvC, mutL, and nfi genes are knocked out.

In some examples, the sbcC gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 1. In other examples, the sbcC gene comprises the nucleotide sequence as set forth in SEQ ID NO: 1.

In some examples, the sbcD gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 2. In other examples, the sbcD gene comprises the nucleotide sequence as set forth in SEQ ID NO: 2.

In some examples, the nfi gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 3. In other examples, the nfi gene comprises the nucleotide sequence as set forth in SEQ ID NO: 3.

In some examples, the mutL gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 4. In other examples, the mutL gene comprises the nucleotide sequence as set forth in SEQ ID NO: 4.

In some examples, the ruvA gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 5. In other examples, the ruvA gene comprises the nucleotide sequence as set forth in SEQ ID NO: 5.

In some examples, the ruvB gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 6. In other examples, the ruvB gene comprises the nucleotide sequence as set forth in SEQ ID NO: 6.

In some examples, the ruvC gene comprises a nucleotide sequence having at least 90% (e.g., at least 91%, or at least 92%, or at least 93%, or at least 94%, or at least 95%, or at least 96%, or at least 97%, or at least 98%, or at least 99%) similarity to SEQ ID NO: 7. In other examples, the ruvC gene comprises the nucleotide sequence as set forth in SEQ ID NO: 7.

In some examples, the host cell further comprises a knocked-in lacIq or lacIq1 gene. In other examples, the sbcC, sbcD, and mutL genes in the cells are knocked out, and the cells comprise a knocked-in lacIq gene. In other examples, the sbcC, sbcD, and mutL genes in the cells are knocked out, and the cells comprise a knocked-in lacIq1 gene. In other examples, the sbcC, sbcD, and nfi genes in the cells are knocked out, and the cells comprise a knocked-in lacIq gene. In other examples, the sbcC, sbcD, and nfi genes in the cells are knocked out, and the cells comprise a knocked-in lacIq1 gene. In other examples, the sbcC, sbcD, ruvA, ruvB, and ruvC genes in the cells are knocked out, and the cells comprise a knocked-in lacIq gene. In other examples, the sbcC, sbcD, ruvA, ruvB, and ruvC genes in the cells are knocked out, and the cells comprise a knocked-in lacIq1 gene. In other examples, the sbcC, sbcD, and ruvC genes in the cells are knocked out, and the cells comprise a knocked-in lacIq gene. In other examples, the sbcC, sbcD, and ruvC genes in the cells are knocked out, and the cells comprise a knocked-in lacIq1 gene. In other examples, the sbcC, sbcD, mutL, and nfi genes in the cells are knocked out, and the cells comprise a knocked-in lacIq gene. In other examples, the sbcC, sbcD, mutL, and nfi genes in the cells are knocked out, and the cells comprise a knocked-in lacIq1 gene.

Also provided herein is a host cell in which the sbcC and/or sbcD genes are knocked out, and which further comprises a knocked-in lacIq or lacIq1 gene. In some examples, the sbcC and sbcD genes in the cell are knocked out, and the cell comprises a knocked-in lacIq gene. In other examples, the sbcC and sbcD genes in the cell are knocked out, and the cell comprises a knocked-in lacIq1 gene. In other examples, the sbcC gene in the cell is knocked out, and the cell comprises a knocked-in lacIq gene. In other examples, the sbcC gene in the cell is knocked out, and the cell comprises a knocked-in lacIq1 gene. In other examples, the sbcD gene in the cell is knocked out, and the cell comprises a knocked-in lacIq gene. In other examples, the sbcD gene in the cell is knocked out, and the cell comprises a knocked-in lacIq1 gene.

In some examples, the nucleotide sequence of the lacIq gene has at least 90% (e.g., at least 91%, or at least 92%, or at least 93%, or at least 94%, or at least 95%, or at least 96%, or at least 97%, or at least 98%, or at least 99%) sequence identity to SEQ ID NO: 8. In other examples, the nucleotide sequence of the lacIq gene is as set forth in SEQ ID NO: 8.

In some examples, the nucleotide sequence of the lacIq1 gene has at least 90% (e.g., at least 91%, or at least 92%, or at least 93%, or at least 94%, or at least 95%, or at least 96%, or at least 97%, or at least 98%, or at least 99%) sequence identity to SEQ ID NO: 9. In other examples, the nucleotide sequence of the lacIq1 gene is as set forth in SEQ ID NO: 9.

In some examples, the host cell is an *E. coli* cell. In other examples, the *E. coli* cell includes, but is not limited to, Top10, JM108, or NEB Stable cell, etc.

In another aspect, this application provides use of the above host cell in the preparation of a nucleotide sequence comprising a hairpin structure. In some embodiments, provided is use of the above host cell in the cloning or expression of a nucleotide sequence comprising a hairpin structure.

In some embodiments, the nucleotide sequence comprising a hairpin structure may be a DNA sequence comprising one or more hairpin structures. In other embodiments, the nucleotide sequence comprising a hairpin structure may be a DNA sequence comprising two or more hairpin structures. In some examples, the DNA sequence may be a plasmid. In some embodiments, the nucleotide sequence comprising a hairpin structure may be an RNA sequence comprising one or more hairpin structures.

In some examples, the hairpin structure is an ITR sequence. In other examples, the hairpin structure is an ITR derived from an adeno-associated virus (AAV). In other examples, the number of nucleotides in the A region in the ITR sequence is at least 20. For example, the number of nucleotides in the A region in the ITR sequence is at least 25, or at least 28, or at least 30, or at least 32, or at least 34, or at least 36, or at least 38, or at least 40. In some preferred examples, the number of nucleotides in the A region in the ITR sequence is at least 40.

In some examples, the hairpin structure sequence cloned or expressed in the host cell has stability, including replication stability and/or passage stability. In other examples, the hairpin structure sequence cloned or expressed in the host cell has replication stability. In other examples, the hairpin structure sequence cloned or expressed in the host cell has passage stability. In other examples, the hairpin structure sequence cloned or expressed in the host cell has replication stability and passage stability. In some examples, the hairpin structure sequence cloned in the host cell has replication stability and passage stability.

The inventors have surprisingly found that the knockin or knockout of several genes can further improve the stability of the hairpin structure in the sbcC or sbcC-sbcD-knockout host cell. In particular, 1) the knockin of lacIq improves the stability of the hairpin structure in the sbcC or sbcC-sbcD-knockout host cell; 2) the knockin of lacIq1 improves the stability of the hairpin structure in the sbcC or sbcC-sbcD-knockout host cell; 3) the knockout of mutL improves the stability of the hairpin structure in the sbcC or sbcC-sbcD-knockout host cell; 4) the knockout of ruvA, ruvB, and/or ruvC improves the stability of the hairpin structure in the sbcD or sbcC-sbcD-knockout host cell; and 5) the knockout of mutL and the knockin of lacIq1 improves the stability of the hairpin structure in the sbcC or sbcC-sbcD-knockout host cell.

The technical solutions of the present invention will be further described in detail below through examples and in combination with the accompanying drawings. Unless otherwise specified, the materials in the examples described below are all conventional products that are commercially available. Those skilled in the art to which the present invention belongs will understand that the methods and materials described below are exemplary only and should not be construed as limiting the scope of the present invention.

### Example 1: Construction of strain mutants

### 1.1. Knockout and knockin of strain genes:

Related genes were knocked out in the genomes of Top10, JM108, and NEB Stable using CRISPR-Cas9 technology. Exemplarily, in the Top10 strain, the sequences of the knocked-out genes are as shown below: the sbcC gene (SEQ ID NO: 1), the sbcD gene (SEQ ID NO: 2), the nfi gene (SEQ ID NO: 3), the mutL gene (SEQ ID NO: 4), the ruvA gene (SEQ ID NO: 5), the ruvB gene (SEQ ID NO: 6), and the ruvC gene (SEQ ID NO: 7); and the sequences of the knocked-in genes are exemplarily as shown below: the lacIq gene (SEQ ID NO: 8) and the lacIq1 gene (SEQ ID NO: 9).

1.1.1. The sequences of the genes to be knocked out were uploaded to the sgRNA design website (https://www.atum.bio/eCommerce/cas9/input), and sgRNAs with a length of 20 bp were designed. The 300-bp sequence near the location of the lacIq or lacIq1 gene to be knocked in was also uploaded to the sgRNA design website, and sgRNAs with a length of 20 bp were designed. For the sequence information, see the targeting sequences of sgRNAs (SEQ ID NOs: 11-19).

1.1.2. The sgRNAs (SEQ ID NOs: 11-19) and the corresponding donor sequences (SEQ ID NOs: 22-30) were constructed into all-in-one plasmids, and the plasmid maps of some plasmids are shown in FIGs. 2A-2F.

1.1.3. The pCas plasmid (the plasmid map is shown in FIG. 2G) was transformed into each of Top10, JM108, or NEB Stable chemically competent cells by chemical transformation. The transformed cells were spread onto LB solid kanamycin-resistant plates, and the plates were inverted and incubated at 30°C. Positive clones were obtained as verified by colony PCR (the PCR primers are shown in Table 1) and Sanger sequencing. Then, the all-in-one plasmids were electrotransformed into the above three competent cell lines harboring pCas9. The cells in the transformed plates were spread onto LB solid plates supplemented with two antibiotics-kanamycin and spectinomycin, and the plates were inverted and incubated at 30°C. Single colonies were picked, and positive clones were identified via colony PCR (the PCR primers are shown in Table 1).

**Table 1. Primer sequences for colony PCR**

| Sequence number SEQ ID NO: | Primer ID | Sequence |
|---|---|---|
| 31 | pCas-F | GCATGACACCGGACATTATCCTGCAGC |
| 32 | pCas-R | TCCCCAAATACAAAACCAATTTCAGCC |
| 33 | Δ*sbcC-sbcD* KO-F | CGGAATGCCCAGCAAATTGGCGACTGTCAT |
| 34 | Δ*sbcC-sbcD* KO-R | CACCAGCTCCTTCGGCGAAAACGGCTTGGT |
| 35 | Δ*mutL* KO-F | CAACAGTGAAATGGCCAGCTGGCTG |
| 36 | Δ*mutL* KO-R | CGTCAGTTCCGTTAAAGTTTTACCCGAAATGA |
| 37 | Δ*nfi* KO-F | TCACTTTGTATCTGAATGCGCAGATTAAAGC |
| 38 | Δ*nfi* KO-R | TTGCTCTACAGCTGGGTTTTCTTTAAGCTCTT |
| 39 | *ΔruvABC* KO-F | GTTTGTTGATGGTGTGAAATT |
| 40 | Δ*ruvABC* KO-R | CAACCGCAACCGTACCGTTG |
| 41 | *lacIq* KI-F | CCAGCACCCGCCAGGAAACCGGGCG |
| 42 | *lacIq* KI-R | GCTTTATGCACTATCGCAATGTGG |
| 43 | *lacIq*1 KI-F | CCAGCACCCGCCAGGAAACCGGGCGTTTCA |
| 44 | *lacIq*1 KI-R | GGCGCGAGGTGCCGATCACGAAACTACCAG |

1.1.4. Successfully edited positive clones were obtained by colony PCR and Sanger sequencing. Both pCas and all-in-one resistance plasmids used for editing were removed as follows. 0.5 mM IPTG inducer was added to the bacterial culture of positive clones, and the culture was incubated overnight to remove the spectinomycin-resistant all-in-one plasmid; and after verification of successful removal, the bacterial culture was incubated overnight at 37°C to remove the kanamycin-resistant pCas plasmid. The strains with the above genes knocked out/knocked in were prepared into chemically competent cells for future use.

### 1.2. Stability testing of ITR plasmids in mutants

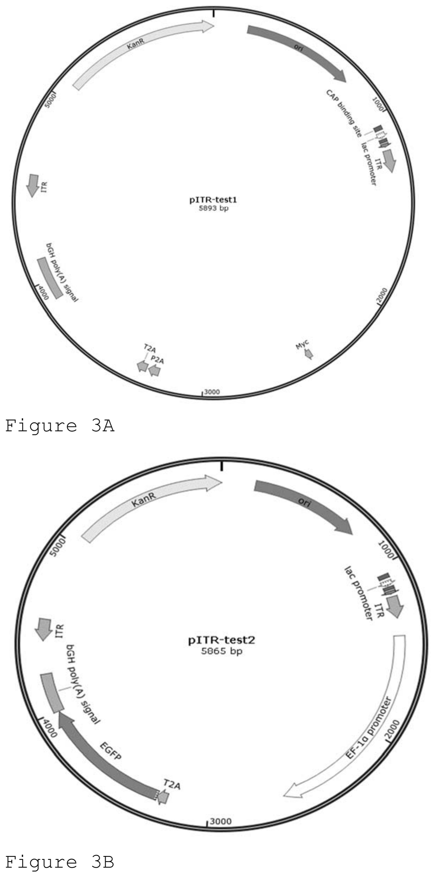

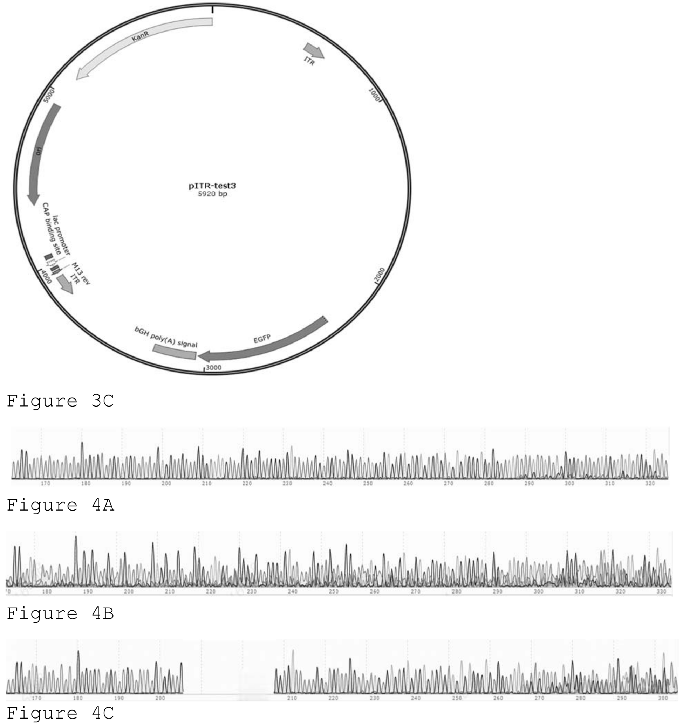

Three plasmids containing a wild-type or modified ITR sequence (SEQ ID NOs: 20-21) derived from the adeno-associated virus (AAV) genome were selected, with pITR-test1, pITR-test2, and pITR-test3 as examples (the plasmid maps are shown in FIGs. 3A-3C). The plasmid construction workflow was as follows. The ITR region sequences of the above three plasmids were obtained by annealing and splicing, and then assembled into the pUC57 kanamycin-resistant vector (vector size: 2579 bp, from GenScript Biotech Co., Ltd.).

Positive clones were selected for sequencing. The three test plasmids with correct sequences as confirmed by sequencing were each transformed into the strains obtained in 1.1.4. as well as a commercial strain such as NEB Stable. Eight clones were picked from each transformed plate and incubated overnight in LB liquid medium at 37°C and 220 rpm. These bacteria were designated as the first generation. The plasmids were extracted from the first-generation bacterial cultures. By using the first-generation bacterial cultures as seed inocula, the second-generation bacteria were prepared by inoculating them at 1 : 1000 and incubating overnight. The resulting bacteria were continuously passaged up to the fourth generation, and the plasmids were extracted for verification of ITR integrity.

Verification method I: The integrity of the ITR regions was verified by Sanger sequencing. Intact ITR was defined as having no deletion, with Sanger sequencing background noise in the ITR region lower than 20% of the main signal (FIG. 4A), deleted ITR was defined as having sequence deletions in the ITR region (FIG. 4C), and impure ITR was defined as having no deletion in ITR, with Sanger sequencing background noise in the ITR region higher than 20% of the main signal (FIG. 4B). The determination examples are as shown in FIGs. 4A-4C.

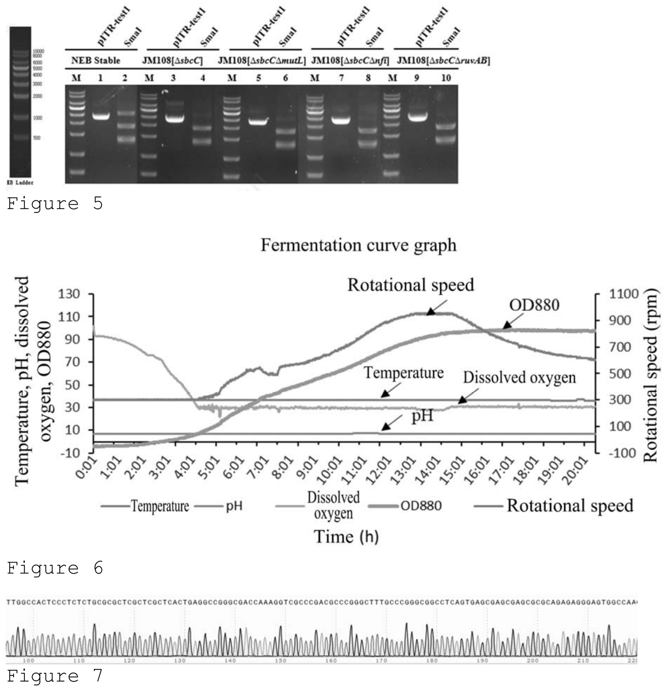

Verification method II: The plasmid size was verified by SmaI restriction digestion, and the proportion of incompletely digested fragments was analyzed by GIS. The sizes of the bands in DNA gel electrophoresis after Smal restriction digestion were consistent with the theoretical values, and the proportion of incomplete digestion was less than 15% (lanes 4, 6, 8, and 10 in FIG. 5), under which circumstances, the restriction digestion was determined to have passed; and for other cases of restriction digestion, the restriction digestion was determined to have failed (lane 2 in FIG. 5).

In the example of the present invention, one plasmid with a correct sequence as confirmed by sequencing was randomly selected from each of the three test plasmids and transformed into the strains obtained in 1.1.4. Eight clones were picked from each transformation, resulting in a total of 24 clones. These clones were each subjected to four consecutive generations of passage and testing. The first- and fourth-generation passage clones were selected, from which plasmids were extracted, and the ITR integrity ratio was determined by Sanger sequencing and SmaI restriction digestion. By summarizing the ITR integrity ratio data from these passage clones, the stability of different mutant hosts in replicating the ITR structure is determined. Higher Smal restriction digestion pass rate and higher integrity ratio of the ITR structure by Sanger sequencing indicate higher stability of the mutant host in replicating the ITR structure.

### Example 2: Knockin of lacIq gene improves replication stability of ITRs in E. coli

Strains with genotypes as shown in Table 2 were constructed using the method described in Example 1, in which the ITR plasmids contained the LacP promoter element. The effect of mutations in *E. coli* on the replication stability of the ITR plasmids was verified through the continuous passage experiment described in Example 1.2. Table 2 summarizes the ITR integrity ratio data of the three test plasmids in the first- and fourth-generations strains. In the commercial NEB Stable strain, the ITR integrity ratio in 24 passage clones of the first generation was shown to be 12.5% by SmaI restriction digestion-based screening and Sanger sequencing. In the fourth-generation plasmids, the deletion ratio of the ITR regions was shown to be 100% by Sanger sequencing, and the SmaI restriction digestion pass rate was 0%. In the fourth-generation plasmids of strains with sbcC or sbcC and sbcD knocked out, the SmaI restriction digestion pass rates and the ITR integrity ratios as determined by Sanger sequencing were higher than those in the commercial NEB Stable strain, indicating that the knockout of the sbcC gene alone or the sbcC and sbcD genes in *E. coli* has a positive effect on the replication stability of ITRs. Notably, in the JM108 [ΔsbcC-sbcD lacIq], Top10 [ΔsbcC-sbcD lacIq], and NEB Stable [ΔsbcC-sbcD lacIq] strains obtained by further knocking in the lacIq gene on the basis of strains with sbcC and sbcD knocked out, the SmaI restriction digestion pass rates and the ITR integrity ratios as shown by Sanger sequencing in the first generation were 62.5%, 70.8%, and 83.3%, respectively. By the fourth generation, the ITR integrity ratios remained at an ITR integrity ratio level of 50% or more, indicating that the knockin of the lacIq gene further improves the replication stability of the ITR plasmids in *E. coli* host cells.

**Table 2. Stability of ITR plasmids in commercial and mutant strains**

| Strain | First generation | | | | Fourth generation | | | |
|---|---|---|---|---|---|---|---|---|
| | Smal restriction digestion pass rate | Intact ITR | Delete d ITR | Impur e ITR | Smal restriction digestion pass rate | Intact ITR | Delete d ITR | Impur e ITR |
| NEB Stable | 12.5% | 12.5% | 83.3% | 4.2% | 0.0% | 0.0% | 100.0 % | 0.0% |
| JM108 [Δ*sbcC*] | 41.7% | 41.7% | 33.3% | 25.0% | 33.3% | 33.3% | 50.0% | 16.7% |
| JM108 [Δ*sbcC-sbcD*] | 45.8% | 45.8% | 25.0% | 29.2% | 29.2% | 29.2% | 41.7% | 29.2% |
| JM108 [Δ*sbcC-sbcD lacIq*] | 62.5% | 62.5% | 20.8% | 16.7% | 54.2% | 54.2% | 25.0% | 20.8% |
| Top10 [Δ*sbcC*] | 37.5% | 37.5% | 37.5% | 25.0% | 29.2% | 29.2% | 45.8% | 25.0% |
| Top10 [Δ*sbcC-sbcD*] | 41.7% | 41.7% | 25.0% | 33.3% | 29.2% | 29.2% | 41.7% | 29.2% |
| Top10 [Δ*sbcC-sbcD lacIq*] | 70.8% | 70.8% | 12.5% | 16.7% | 58.3% | 58.3% | 25.0% | 16.7% |
| NEB Stable [Δ*sbcC*] | 33.3% | 33.3% | 41.7% | 25.0% | 29.2% | 29.2% | 45.8% | 25.0% |
| NEB Stable [Δ*sbcC-sbcD*] | 41.7% | 41.7% | 25.0% | 33.3% | 29.2% | 29.2% | 41.7% | 29.2% |
| NEB Stable [Δ*sbcC-sbcD lacIq*] | 83.3% | 83.3% | 8.3% | 8.3% | 62.5% | 62.5% | 20.8% | 16.7% |

### Example 3: Knockin of lacIq1 gene improves replication stability of ITRs in E. coli

Strains with genotypes as shown in Table 3 were constructed using the method described in Example 1, in which the ITR plasmids contained the LacP promoter element. The effect of mutations in *E. coli* on the replication stability of the ITR plasmids was verified through the continuous passage experiment described in Example 1.2.

Table 3 summarizes the ITR integrity ratio data of the three test plasmids in the first- and fourth-generations strains. In contrast, in the three strains JM108 [ΔsbcC-sbcD lacIq1], Top10 [ΔsbcC-sbcD lacIq1], and NEB Stable [ΔsbcC-sbcD lacIq1] obtained by further knocking in the lacIq1 gene on the basis of strains with sbcC or sbcC and sbcD knocked out, the ITR integrity ratios in the first generation were shown to be 62.5%, 75.0%, and 83.3% by Smal restriction digestion-based screening and Sanger sequencing. By the fourth generation, the ITR integrity ratios were 50.0%, 62.5%, and 66.7%, respectively. In conclusion, with the sbcC gene or the sbcC and sbcD genes knocked out, the integrity ratios of the ITR regions were higher than that in the commercial NEB Stable strain, indicating a certain effect on maintaining the ITR stability. With further knocking in the lacIq1 gene on the basis of knocking out the sbcC-sbcD genes, the ITR integrity ratios remained at a level of 50% or more as the passage time was prolonged, indicating that the knockin of the lacIq1 gene in the strains has a promoting effect on maintaining the ITR stability.

**Table 3. Stability of ITR plasmids in commercial and mutant strains**

| Strain | First generation | | | | Fourth generation | | | |
|---|---|---|---|---|---|---|---|---|
| | Smal restriction digestion pass rate | Intact ITR | Delete d ITR | Impur e ITR | Smal restriction digestion pass rate | Intact ITR | Delete d ITR | Impur e ITR |
| NEB Stable | 12.5% | 12.5% | 83.3% | 4.2% | 0.0% | 0.0% | 100.0 % | 0.0% |
| JM108 [Δ*sbcC*] | 41.7% | 41.7% | 33.3% | 25.0% | 33.3% | 33.3% | 50.0% | 16.7% |
| JM108 [Δ*sbcC-sbcD*] | 45.8% | 45.8% | 25.0% | 29.2% | 29.2% | 29.2% | 41.7% | 29.2% |
| JM108 [Δ*sbcC-sbcD lacIq*1] | 62.5% | 62.5% | 20.8% | 16.7% | 50.0% | 50.0% | 29.2% | 20.8% |
| Top10 [Δ*sbcC*] | 37.5% | 37.5% | 37.5% | 25.0% | 29.2% | 29.2% | 45.8% | 25.0% |
| Top10 [Δ*sbcC-sbcD*] | 41.7% | 41.7% | 25.0% | 33.3% | 29.2% | 29.2% | 41.7% | 29.2% |
| Top10 [Δ*sbcC-sbcD lacIq*1] | 75.0% | 75.0% | 16.7% | 8.3% | 62.5% | 62.5% | 20.8% | 16.7% |
| NEB Stable [Δ*sbcC*] | 33.3% | 33.3% | 41.7% | 25.0% | 29.2% | 29.2% | 45.8% | 25.0% |
| NEB Stable [Δ*sbcC-sbcD*] | 41.7% | 41.7% | 25.0% | 33.3% | 29.2% | 29.2% | 41.7% | 29.2% |
| NEB Stable [Δ*sbcC-sbcD lacIq*1] | 83.3% | 83.3% | 12.5% | 4.2% | 66.7% | 66.7% | 16.7% | 16.7% |

### Example 4: Knockout of ruvABC gene improves replication stability of ITRs in E. coli

Strains with genotypes as shown in Table 4 were constructed using the method described in Example 1. The effect of mutations in *E. coli* on the replication stability of the ITR plasmids was verified through the continuous passage experiment described in Example 1.2.

Table 4 summarizes the ITR integrity ratio data of the three test plasmids in the first- and fourth-generations strains. In contrast, in the JM108 strains obtained by further knocking out the ruvC, ruvAB, or ruvABC gene on the basis of strains with sbcC or sbcC and sbcD knocked out, the ITR integrity ratios in the first generation were shown to be 58.3%, 54.2%, and 54.2%, respectively, by SmaI restriction digestion-based screening and Sanger sequencing; in the Top10 and NEB Stable strains obtained by further knocking out the ruvABC gene on the basis of knocking out sbcC and sbcD, the Smal restriction digestion pass rates and the ITR integrity ratios as shown by Sanger sequencing in the first generation were 62.5% and 66.7%, respectively; and in the three strains obtained by further knocking out the ruvC, ruvAB, or ruvABC gene on the basis of knocking out sbcC and sbcD, the integrity ratios of the plasmid ITR regions were all 50% or more by the fourth generation, which were higher than those in strains with sbcC or sbcC and sbcD knocked out and the commercial NEB Stable strain. In conclusion, further knockout of the ruvC, ruvAB, or ruvABC gene on the basis of knocking out the sbcC gene or the sbcC and sbcD genes has a promoting effect on maintaining the ITR stability.

**Table 4. Stability of ITR plasmids in commercial and mutant strains**

| Strain | First generation | | | | Fourth generation | | | |
|---|---|---|---|---|---|---|---|---|
| | Smal restriction digestion pass rate | Intact ITR | Delete d ITR | Impur e ITR | Smal restriction digestion pass rate | Intact ITR | Delete d ITR | Impur e ITR |
| NEB Stable | 12.5% | 12.5% | 83.3% | 4.2% | 0.0% | 0.0% | 100.0 % | 0.0% |
| JM108 [Δ*sbcC*] | 41.7% | 41.7% | 33.3% | 25.0% | 33.3% | 33.3% | 50.0% | 16.7% |
| JM108[Δ*sbcC-sbcD*] | 45.8% | 45.8% | 25.0% | 29.2% | 29.2% | 29.2% | 41.7% | 29.2% |
| JM108 [Δ*sbcD-*Δ*ruvC*] | 58.3% | 58.3% | 33.3% | 8.3% | 54.2% | 54.2% | 33.3% | 12.5% |
| JM108[Δ*sbcC-sbcD* Δ*ruvAB*] | 54.2% | 54.2% | 20.8% | 25.0% | 50.0% | 50.0% | 25.0% | 25.0% |
| JM108[Δ*sbcC-sbcD* Δ*ruvABC*] | 54.2% | 54.2% | 29.2% | 16.7% | 50.0% | 50.0% | 33.3% | 16.7% |
| Top10 [Δ*sbcC*] | 37.5% | 37.5% | 37.5% | 25.0% | 29.2% | 29.2% | 45.8% | 25.0% |
| Top10 [Δ*sbcC-sbcD*] | 41.7% | 41.7% | 25.0% | 33.3% | 29.2% | 29.2% | 41.7% | 29.2% |
| Top10 [Δ*sbcC-sbcD* Δ*ruvABC*] | 62.5% | 62.5% | 20.8% | 16.7% | 54.2% | 54.2% | 33.3% | 12.5% |
| NEB Stable [Δ*sbcC*] | 33.3% | 33.3% | 41.7% | 25.0% | 29.2% | 29.2% | 45.8% | 25.0% |
| NEB Stable [*ΔsbcC-sbcD*] | 41.7% | 41.7% | 25.0% | 33.3% | 29.2% | 29.2% | 41.7% | 29.2% |
| NEB Stable [Δ*sbcC-sbcD* Δ*ruvABC*] | 66.7% | 66.7% | 16.7% | 16.7% | 58.3% | 58.3% | 25.0% | 16.7% |

### Example 5: Knockout of mutL gene improves replication stability of ITRs in E. coli

Strains with genotypes as shown in Table 5 were constructed using the method described in Example 1. The effect of mutations in E. coli on the replication stability of the ITR plasmids was verified through the continuous passage experiment described in Example 1.2. Table 5 summarizes the ITR integrity ratio data of the three test plasmids in the first- and fourth-generations strains. In contrast, in the three strains JM108 [ΔsbcC-sbcD ΔmutL], Top10 [ΔsbcC-sbcD ΔmutL], and NEB Stable [ΔsbcC-sbcD ΔmutL] obtained by further knocking out the mutL gene on the basis of strains with the sbcC and sbcD genes knocked out, the ITR integrity ratios in the first generation were shown to be 50.0%, 58.3%, and 66.7%, respectively, by Smal restriction digestion-based screening and Sanger sequencing. By the fourth generation, the ITR integrity ratios remained higher than those in strains with sbcC and sbcD knocked out and the commercial NEB Stable strain. In conclusion, further knockout of the mutL gene on the basis of strains with sbcC and sbcD knocked out has a certain degree of improvement in maintaining the ITR stability.

**Table 5. Stability of ITR plasmids in commercial and mutant strains**

| Strain | First generation | | | | Fourth generation | | | |
|---|---|---|---|---|---|---|---|---|
| | Smal restriction digestion pass rate | Intact ITR | Delete d ITR | Impur e ITR | Smal restriction digestion pass rate | Intact ITR | Delete d ITR | Impur e ITR |
| NEB Stable | 12.5% | 12.5% | 83.3% | 4.2% | 0.0% | 0.0% | 100.0 % | 0.0% |
| JM108 [Δ*sbcC*] | 41.7% | 41.7% | 33.3% | 25.0% | 33.3% | 33.3% | 50.0% | 16.7% |
| JM108 [Δ*sbcC-sbcD*] | 45.8% | 45.8% | 25.0% | 29.2% | 29.2% | 29.2% | 41.7% | 29.2% |
| JM108 [Δ*sbcC-sbcD* Δ*mutL*] | 50.0% | 50.0% | 25.0% | 25.0% | 50.0% | 50.0% | 41.7% | 8.3% |
| Top10 [Δ*sbcC*] | 37.5% | 37.5% | 37.5% | 25.0% | 29.2% | 29.2% | 45.8% | 25.0% |
| Top10 [Δ*sbcC-sbcD*] | 41.7% | 41.7% | 25.0% | 33.3% | 29.2% | 29.2% | 41.7% | 29.2% |
| Top10 [Δ*sbcC-sbcD* Δ*mutL*] | 58.3% | 58.3% | 20.8% | 20.8% | 50.0% | 50.0% | 25.0% | 25.0% |
| NEB Stable [Δ*sbcC*] | 33.3% | 33.3% | 41.7% | 25.0% | 29.2% | 29.2% | 45.8% | 25.0% |
| NEB Stable [Δ*sbcC-sbcD*] | 41.7% | 41.7% | 25.0% | 33.3% | 29.2% | 29.2% | 41.7% | 29.2% |
| NEB Stable [Δ*sbcC-sbcD* Δ*mutL*] | 66.7% | 66.7% | 20.8% | 12.5% | 54.2% | 54.2% | 29.2% | 16.7% |

### Example 6: Knockout of nfi gene improves replication stability of ITRs in E. coli

Strains with genotypes as shown in Table 6 were constructed using the method described in Example 1. The effect of mutations in *E. coli* on the replication stability of the ITR plasmids was verified through the continuous passage experiment described in Example 1.2.

**Table 6. Stability of ITR plasmids in commercial and mutant strains**

| Strain | First generation | | | | Fourth generation | | | |
|---|---|---|---|---|---|---|---|---|
| | Smal restriction digestion pass rate | Intact ITR | Delete d ITR | Impure ITR | Smal restriction digestion pass rate | Intact ITR | Delete d ITR | Impur e ITR |
| NEB Stable | 12.5% | 12.5% | 83.3% | 4.2% | 0.0% | 0.0% | 100.0 % | 0.0% |
| JM108 [Δ*sbcC*] | 41.7% | 41.7% | 33.3% | 25.0% | 33.3% | 33.3% | 50.0% | 16.7% |
| JM108 [Δ*sbcC-sbcD*] | 45.8% | 45.8% | 25.0% | 29.2% | 29.2% | 29.2% | 41.7% | 29.2% |
| JM108 [Δ*sbcC-sbcD* Δ*nfi*] | 58.3% | 58.3% | 25.0% | 16.7% | 54.2% | 54.2% | 29.2% | 16.7% |
| Top10 [Δ*sbcC*] | 37.5% | 37.5% | 37.5% | 25.0% | 29.2% | 29.2% | 45.8% | 25.0% |
| Top10 [*ΔsbcC-sbcD*] | 41.7% | 41.7% | 25.0% | 33.3% | 29.2% | 29.2% | 41.7% | 29.2% |
| Top10 [Δ*sbcC-sbcD* Δ*nfi*] | 66.7% | 66.7% | 25.0% | 8.3% | 54.2% | 54.2% | 33.3% | 12.5% |
| NEB Stable [Δ*sbcC*] | 33.3% | 33.3% | 41.7% | 25.0% | 29.2% | 29.2% | 45.8% | 25.0% |
| NEB Stable[Δ*sbcC-sbcD*] | 41.7% | 41.7% | 25.0% | 33.3% | 29.2% | 29.2% | 41.7% | 29.2% |
| NEB Stable[Δ*sbcC-sbcD* Δ*nfi*] | 79.2% | 79.2% | 12.5% | 8.3% | 70.8% | 70.8% | 20.8% | 8.3% |

Table 6 summarizes the ITR integrity ratio data of the three test plasmids in the first- and fourth-generations strains. In the strains obtained by further knocking out the nfi gene on the basis of strains with the sbcC and sbcD genes knocked out, the ITR integrity ratios in the first generation were shown to be 58.3%, 66.7%, and 79.2% by SmaI restriction digestion-based screening and Sanger sequencing. By the fourth generation, the ITR integrity ratios remained higher than those in the commercial NEB Stable strain and strains with the sbcC and sbcD genes knocked out. With further knocking out the nfi gene on the basis of strains with the sbcC and sbcD genes knocked out, the ITR integrity ratios remained at a level of 50% or more as the passage time was prolonged, demonstrating that the knockout of the nfi gene has a certain effect on maintaining the ITR stability.

### Example 7: Combined knockout of mutL and nfi improves replication stability of ITRs in E. coli

Strains with genotypes as shown in Table 7 were constructed using the method described in Example 1. The effect of mutations in *E. coli* hosts on the replication stability of the ITR plasmids was verified through the continuous passage experiment described in Example 1.2.

**Table 7. Stability of ITR plasmids in commercial and mutant strains**

| Strain | First generation | | | | Fourth generation | | | |
|---|---|---|---|---|---|---|---|---|
| | Smal restriction digestion pass rate | Intact ITR | Delete d ITR | Impur e ITR | Smal restriction digestion pass rate | Intact ITR | Delete d ITR | Impur e ITR |
| NEB Stable | 12.5% | 12.5% | 83.3% | 4.2% | 0.0% | 0.0% | 100.0 % | 0.0% |
| JM108 [Δ*sbcC*] | 41.7% | 41.7% | 33.3% | 25.0% | 33.3% | 33.3% | 50.0% | 16.7% |
| JM108[Δ*sbcC*Δ*mut L*Δ*nfi*] | 62.5% | 62.5% | 20.8% | 16.7% | 58.3% | 58.3% | 29.2% | 12.5% |
| Top10[Δ*sbcC*Δ*mut L*Δ*nfi*] | 66.7% | 66.7% | 16.7% | 16.7% | 66.7% | 66.7% | 25.0% | 8.3% |
| NEB Stable[Δ*sbcC*Δ*mut L*Δ*nfi*] | 62.5% | 62.5% | 12.5% | 25.0% | 54.2% | 54.2% | 25.0% | 20.8% |

Table 7 summarizes the ITR integrity ratio data of the three test plasmids in the first- and fourth-generations strains. In the commercial NEB Stable strain, the ITR integrity ratio in the fourth-generation passage clones was zero, that is, the entire ITR region was deleted, and the stability of the strain was relatively poor. However, in the JM108 strain with the sbcC gene knocked out, the ITR integrity ratio was 33.3% by the fourth generation, and no problem of entire ITR deletion was observed. In contrast, in strains obtained by sequentially knocking out the mutL and nfi genes on the basis of the sbcC-knockout strains, the ITR integrity ratios in the first generation were 62.5%, 66.7%, and 62.5%. By the fourth generation, the ITR integrity ratios remained at a level of 50% or more as the passage time was prolonged, which were higher than those in strains with sbcC knocked out and the commercial NEB Stable strain, demonstrating that the knockout of both mutL and nfi genes has a promoting effect on maintaining the ITR stability.

### Example 8: Fermentation of JM108 [ΔsbcC-sbcD ΔmutL lacIq] strain

Strains with genotypes as shown in Table 8 were constructed using the method described in Example 1. The effect of mutations in *E. coli* strains on the fermentation stability of the ITR plasmids was verified through the continuous passage experiment described in Example 1.2. The pITR-test1 test plasmid with an intact ITR region (the plasmid map is shown in FIG. 3A) was transformed into each of the commercial NEB Stable strain, as well as the JM108[ΔsbcC] and JM108[ΔsbcC-sbcD ΔmutL lacIq] strains. The plasmid GenS-pUC57-AAV-NoTer (see Example 8 of Patent Application CN 202311099203.6) was inserted into the eukaryotic sequence region of the pITR-test1 plasmid via homologous recombination. The modified plasmid, which had the same final size as pITR-test1, was named GenS-pUC57-AAV-test1. The integrity of the ITR plasmids was evaluated using high-density *E. coli* fermentation technology. Table 8 summarizes the results of the metrics such as OD600, plasmid yield, Smal restriction digestion-based screening, and Sanger sequencing. The OD600 values and plasmid yields of JM108[ΔsbcC] and JM108[ΔsbcC-sbcD ΔmutL lacIq] were nearly identical to those of the commercial NEB Stable strain, and the OD600 value and plasmid yield of the JM108[ΔsbcC-sbcD ΔmutL lacIq] strain containing GenS-pUC57-AAV-test1 were even higher.

The ITR integrity ratios in the mutated strains were shown to be higher than that in the commercial NEB Stable strain by Smal restriction digestion-based screening and Sanger sequencing, and the ITR integrity ratio in the JM108 [ΔsbcC-sbcD ΔmutL lacIq] strain containing pITR-test1 reached as high as 83.3% after fermentation. Plasmids with complete digestion and 100% ITR integrity were obtained by combining the modified JM108 [ΔsbcC-sbcD ΔmutL lacIq] strain with the modified vector GenS-pUC57-AAV-test1. This indicates that compared with the prior art, the strain has the characteristics of high yield in plasmid production and more intact ITR regions. The corresponding fermentation curve graph of the JM108 [ΔsbcC-sbcD ΔmutL lacIq] strain containing pITR-test1 is shown in FIG. 6, and the Sanger sequencing chromatogram is shown in FIG. 7. The sequencing results showed that the ITR region was 100% identical to the sequence of SEQ ID NO: 20.

**Table 8. Fermentation evaluation of JM108[ΔsbcC-sbcD ΔmutL lacIq] strain**

| Strain | Fermentation mode | Vector | OD600 at harvest | Plasmid yield (post-MN purification yield) | Smal restriction digestion pass rate | Intact ITR | Deleted ITR | Impure ITR |
|---|---|---|---|---|---|---|---|---|
| NEB Stable | Genscript high density fermentation | pITR-test1 | 48 | 205 mg/L | 8.3% | 8.3% | 91.7% | 0.0% |
| JM108 [Δ*sbcC*] | Genscript high density fermentation | pITR-test1 | 46 | 203 mg/L | 66.7% | 66.7 % | 16.7% | 16.7% |
| JM108 [Δ*sbcC-sbcD* Δ*mutL lacIq*] | Genscript high density fermentation | pITR-test1 | 50 | 220 mg/L | 83.3% | 83.3 % | 8.3% | 8.3% |
| JM108 [Δ*sbcC-sbcD* Δ*mutL lacIq*] | Genscript high density fermentation | GenS-pUC57 -AAV-test 1 | 54 | 256mg/L | 100% | 100% | 0% | 0% |

### Example 9: Supercoil ratios of plasmids extracted from mutant hosts

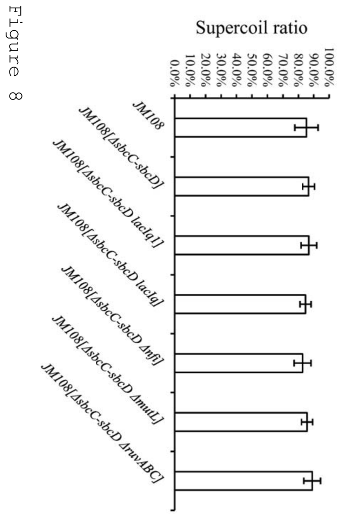

Five different types of plasmids were selected and each transformed into the commercial JM108 strain, as well as the JM108 [ΔsbcC-sbcD], JM108[ΔsbcC-sbcD lacIq1], JM108 [ΔsbcC-sbcD lacIq], JM108 [ΔsbcC-sbcD Δnfi], JM108 [ΔsbcC-sbcD ΔmutL], and JM108 [ΔsbcC-sbcD ΔruvABC] strains. Single colonies were randomly selected for continuous passage experiments. Plasmids were extracted from the first- and fourth-generation strains. 200 ng of the plasmids was subjected to DNA gel electrophoresis, and the supercoil ratios of the plasmids were analyzed using the fully automated gel imaging analysis system GIS300. The corresponding average supercoil ratio for each strain is shown in FIG. 8. Unexpectedly, the supercoil ratios of the plasmids obtained from the mutated strains were nearly identical to that of JM108, all of which were greater than 82%.

In summary, on the basis of strains with the sbcC and/or sbcD genes knocked out, the respective editing of the lacIq, lacIq1, mutL, nfi, ruvAB, or ruvC gene further improves the stability of ITR plasmids in the mutant hosts. In particular, when the structural complexity of the ITRs is high (with the A-A' region being 43 bp), by using the strain of the present invention, the replication stability of ITR-containing plasmids, with increasing passage, is superior to that of commercial strains.

The different ITR sequences and the different plasmids used in the experiments were not different in stability in the host cells of the present invention (data not shown).

The embodiments of the present invention are not limited to the above examples, and various modifications and improvements in forms and details may be made to the present invention by those of ordinary skill in the art without departing from the spirit and scope of the present invention, and these modifications and improvements are all considered to fall within the scope of protection of the present invention.

The nucleotide sequences referred to herein are as follows.
*E. coli* sbcC gene sequence 
TTATTTCACTGCAAACGTACTTTCCAGTTTGCTGTAGCCCAGGCCG
TTGATCTTTTTCACTTTGATCTGCACCGGAATACGCTCTTTCATCGC
TTCTACGTGGCTAATCACACCGATGGTTTTGCCACTGGCGTTCAGG
GCATCCAGCGCATCAAGGGCGGTATCCAGCGTTTCGCTATCCAGCG
TGCCAAAACCTTCATCAAGGAACAGCGAGTCAATACGTGTTTTATG
GCTGACCAGATCCGAAAGCGCCAGCGCCAGCGCCAGACTAACGAG
GAAACTTTCGCCGCCGGAAAGGGTACGGGTATCGCGTACCGCATC
TGCCTGCCAGGTATCAACAACCTCGACTTCCAGCGCCTCGCTGGCT
TTGCGCTGTAACAGATAGCGCCCGTGCAGCCGGGTAAGTTGCTGAT
TAGCGAGATGGACTAAATTATCCAGCGTCAGCCCCTGGGCAAACT
TGCGGAATTTATCGCCCTCTTTGGAACCTATTAGCGAATTCAGATA
TCCCCAGTCCTCAACCTGCTGCGTCATTTGAGCAATTTGCTGCATT
AAGGTTTGTTGTTGCTGACGGTTATCTGCATCCTGCTTCAGCTGCTG
GCGAATCTCGCCTTGACTCGTGGTGTTTTCACGCAACTTTTGGTGA
GTTTGCGCTAACTCTTGCTGAATCTGCTCCACCGTCACAGTGAGAG
CCAACCCGTCGTCAGGTCGGTGTTGTTGATGCTGTGCCAGCGTTTC
TGCTGTCTGAGTGACCAGAGTTTGCGCCTGACGGCGCTGGTTTTCC
AGATTCTGCTTGAGCTGTTCCAGCTGCGTTAGTGTTTGTTCATCCAT
TAGCGCCGCAAGGAACGCCTGCTGATCGTCAAAGACGCTGGCCTG
 
TAGCGCGGTGTCAAACTGCGCCTGGGCTTTTTGCAGACTTTGCGCC
GCCAGAACATCCTGTTGCTGTAACGTCTGCTGCTGGCTGTGTAATG
CGAGACATTGTTCATGTACCTGCCGCCAGTTTTCCAATACCACAGT
TTCTTCGCAGTGCGGGAGTTCATCACTTTGCGGCAACGTTTCCAGA
ATCGGCGTCAGCTGCTGAATACGGTTTTGCAGCGCGGTTAATTCGT
TCTGGCGTTGCTGCCAGCTCTGCGCTTCTTGCTGACGTGTCGCCAA
CCAGCTCTCTTCTTCATCTTCCTGTGGCAATGTCAGTGCATAACCCG
TCAATGTCGTTAAAAGTAGTTGCTGGCGTTGTTCAATTTGCTGTTG
ATACTGGATAATTTGCTGATTATGCGCGGCAATCTGCCCTTGTAAT
TCATGCCGTTGGCTGAGTAACCGCAGCTGGCGTTCGTGCTCATCTT
GTGCATCCAGCCACGGTTGAATATCGTCCAGTGGCTGCAAGGTGAT
ATTGAGGCTGGCCGTGACGGCTTGCCATTGTTGAGTAAGTGCTTGC
TCATCTTGTCGGAGGCTTTGCGCTTCGTTTTCATCACGCTGAAGCTG
CTTTGTTATGGCGTCCAGTTGCCCACGTAGCGTCGCACCTTCTTCAC
CGAGCTTTTTAACTTCGTTTTCCAGCGCCAGTAATCGAGACTGATT
AACGCCAGGCTCCAGCGCCTGATACGCCTCGACCGCCGGGTGGCT
GGTGGAACCACAAAGTGGGCAAGGCTGACCCGCCTGTAACTGTGC
ACGTTGAGCTTCCAGCGTTTTGATGCGCGCTTCCTGCTCGCAAATG
GTTTTCACATCGGCAAGTTGCTGCGTCTTTTCTTTATAACGCTGGCG
CATTTCGTTAAGTGCGGCGTTACGTTGCGTCTGTTCTTGCGTGACAT
TCTGGATAGCGACCTGTAACTGCGCCAGACGTTTTTGTTGGGGAAC
AATCTGTCCATGCAGCGCGACCAGGTGCTGACGCAGTGGGCGTTG
CTCAGCATGTTGCGCCAGGGCGGTAGCAACTTCATCGGCGGTTAAC
GTCAACGTGATCGCCGCAAGCGCATTAAGTTTTTGCTCAGCATGGG
TTAACTGTTGCTGCCATTGCCGCAGATGCTCGCGATCGCTGGTTTG
TTGGGAGAACTGCGCACGCCAACCCGCCGGTTCGTTGTTCCACTGA
CGGAAGCGGTCGTGTTCCTGTAACCAGGTATTCAGGCTTTGTTGCT
GCTGCTGTAATTCTGCTGACTGCTTCGCCGCGTGGTGGCGAATGCT
CGCGCGAAGCGCCATTGTGCTCTGTAAGCGAGTATTTACTTCTTCA
ATCTGCTGGCGAATATGCGCCAGCGCCGCGCTGTGTTCTGCGATGC
GTTCCCAGTGTGGACGAAGATTTCGTGCCGGTTGTGCCAGACTAAG
CGCCGCCAGTTGAGGTTGCGCTTTTTCTTCTTCGGCTAACGCCTGTT
GCAAGGCCTGCTGACGGCGGCTGGCTTCTTGCTGCAATTCGTCCTG
ACGCGTTAACCAGTTTAGCGATTGTTGTTCTTGCTGCTGCGCGGTA
 
ATTAACTGTTTTTCTTCGTCAGTAAGTACCTGCAAACTCGCTGTCA
GCGATTGCACTTGTTCCGGCGTGAGCAACGTGACGCCGCTGGCCTG
CGCTTGCAGCTTCTCCAGCTCTGTGCGGGCCGATTTGTGCTGCTCA
AAAACCATCGCCGAGATTTGCCCGTAGATTTCAGTGCCGGTTAACT
CCTCGAGCAATTCCGCGCGTTCTTTGGGTTTGGCATTCAGGAAGGC
AGCAAATTGCCCCTGCGAAAGCAGCATCGAACGGGTGAAGCGCCC
GTAATCCAGCCCGGTTAACGTCGCTGTCAGTTCCAGCTTATCTTTC
ACTTTGTCGGCGAGAATTTTGCCGTCGGCGCAGCGCGCCAGCTCTA
CGCGTGGCACCTGCAAATTACCGTCGGGTTGGTTACGCGCCCGATT
CTGGCTCCAGAATGCACGGTACGCTTCACCTTTCACTTCAAACTCC
ACCTCCGCCAGACATTCGGCGGTATCGCGGGTCATGAGATCATTTT
GCGATTGTGAAACGTTAGAGAGACGCGGAGTTTCGTGATACAGCG
CCAGACAAATGGCGTCCAGCAGGGTGGTTTTCCCCGCACCTGTTGG
GCCGGTAATAGCAAACAGCCCGTTGCTGGCGAACGGCTCGCGGGT
GAAATCAATCTTCCATTCGCCTTTTAATGAGTTCAGGTTTTTCAGGC
GCAGGCTGAGAATTTTCAT3147 (SEQ ID NO: 1)
*E. coli* sbcD gene sequence 
TCATGCTTCGTGTTCTCCGGCGAGGGTATGCAACGTCGTGGTGAAA
AGATGCTGCAGACGTTGCTGCTGCGATTCATCCAGTTCTTCCAGTG
CCAGACGGCGATTGAACACCTCTTCGACGCTGAGTTCGCTGAGGGT
TTCACGCTGTTGGCTGGCTAACACACGCTCGCGCTGTTCACGACTC
CGACGTACCAGCAATACTTCGACAGGCAATGATTCGGTTAATGCCT
GGATTTTGCGCTGAATATCATGCAGATACTCATCAGTAGTGATTTC
GATATCCAGCCAGACAGGTGGCTCCTGCGATACATCGCGCCACTGT
TCCAGCTGTGCGGTAATCGACGCCAGATCGCCTTTCAGCACTGCCA
TGGGTTGCGTTACCGGTACGTTCAGGTTTTCCACGCTCTCTAATTTG
CCGTTTGAAAATGTCACCAGATGGACATATTTACTCTTACCGCATT
CATCAAAACTCAGTGGAATGGGGGAGCCGCAATAGCGAACATGTT
CCATGCCGCCAATAATCTGTGCGCGGTGAATATGCCCGAGCGCGA
TGTAGTCGGCTGGTGGAAAGTTTTGTGCCGGAAACGCGTCCAGCGT
GCCAATATAAATGTCACGCACGGCGTCACTTTTACTGGCCCCCACG
GTCGTTAAATGTCCCGTGGCGATGATGGGCAGAGGCTGATCGCCG
CGCAGTTTGCAGGCATCGGCATAGTGTTGTTGGTAATAATCGGTAA
TCGCTGCCAGTAAATGCTGCTGTTTTTCAATACCGTTAAGCCCCGC
 
CTGGCTGGTAATAATGTCACGCGGACGTAAAAACGGAATGGGGCA
CAGCACTGCGCCTGGCGTCCCGTCGCGACGAGGCAAGATTTGCGG
CGCATGTCCGGCGCTGGCGACCACGGTAGTATTGAGGAACGCCAT
GATATCGCGCGATTCATTCAGCGTGGCGACCGAGTCATGGTTTCCT
GCCAGTACCACCAGATGACAGCCAGTTTGCTGTAAATTGACAACA
AAACGGTTGTATAACGTGCGGGCGTAACTGGGCGGCGAGCCGGTA
TCGAAAACATCACCGGCAACAATAATCGCATCCACCTGATGGGTTT
GTGCTGTCTCCAGCAGCCAGTCAAGAAAAGCCTGATGTTCAGCTTC
GCGGCTTTTACTGTAGAAGTTCTGGCCGAGATGCCAGTCTGAGGTG
TGAAGGATGCGCAT1203 (SEQ ID NO: 2)
*E. coli* nfi gene sequence 
ATGGATCTCGCGTCATTACGCGCTCAACAAATCGAACTGGCTTCTT
CTGTGATCCGCGAGGATCGACTCGATAAAGATCCACCGGATCTGA
TCGCCGGAGCCGATGTCGGGTTTGAGCAGGGCGGAGAAGTGACGC
GAGCGGCGATGGTGCTGCTGAAATATCCCTCGCTTGAGCTGGTCGA
GTATAAAGTTGCCCGCATCGCCACCACCATGCCTTACATTCCAGGT
TTTCTTTCCTTCCGCGAATATCCTGCGCTGCTGGCAGCGTGGGAGA
TGCTGTCGCAAAAGCCGGATTTAGTGTTTGTCGATGGTCATGGGAT
CTCGCATCCTCGCCGTCTTGGCGTCGCCAGCCATTTTGGCTTATTGG
TGGATGTGCCGACCATTGGCGTGGCGAAAAAACGGCTCTGCGGTA
AATTCGAACCGCTCTCCAGCGAACCGGGCGCGCTGGCCCCACTGA
TGGATAAAGGCGAGCAGCTGGCCTGGGTCTGGCGCAGCAAAGCGC
GCTGTAACCCGTTGTTTATCGCTACCGGCCATCGGGTCAGCGTGGA
CAGCGCGCTGGCGTGGGTACAACGCTGCATGAAAGGCTATCGTCT
GCCGGAGCCAACGCGCTGGGCGGACGCGGTGGCCTCGGAACGTCC
GGCGTTCGTGCGCTATACAGCAAATCAGCCCTAA672 (SEQ ID NO: 3)
*E. coli* mutL gene sequence 
ATGCCAATTCAGGTCTTACCGCCACAACTGGCGAACCAGATTGCCG
CAGGTGAGGTGGTCGAGCGACCTGCGTCGGTAGTCAAAGAACTAG
TGGAAAACAGCCTCGATGCAGGTGCGACGCGTATCGATATTGATA
TCGAACGCGGTGGGGCGAAACTTATCCGCATTCGTGATAACGGCT
GCGGTATCAAAAAAGATGAGCTGGCGCTGGCGCTGGCTCGTCATG
CCACCAGTAAAATCGCCTCTCTGGACGATCTCGAAGCCATTATCAG
CCTGGGCTTTCGCGGTGAGGCGCTGGCGAGTATCAGTTCGGTTTCC
 
CGCCTGACGCTCACTTCACGCACCGCAGAACAGCAGGAAGCCTGG
CAGGCCTATGCCGAAGGGCGCGATATGAACGTGACGGTAAAACCG
GCGGCGCATCCTGTGGGGACGACGCTGGAGGTGCTGGATCTGTTCT
ACAACACCCCGGCGCGGCGCAAATTCCTGCGCACCGAGAAAACCG
AATTTAACCACATTGATGAGATCATCCGCCGCATTGCGCTGGCGCG
TTTCGACGTCACGATCAACCTGTCGCATAACGGTAAAATTGTGCGT
CAGTACCGCGCAGTGCCGGAAGGCGGGCAAAAAGAACGGCGCTTA
GGCGCGATTTGCGGCACCGCTTTTCTTGAACAAGCGCTGGCGATTG
AATGGCAACACGGCGATCTCACGCTACGCGGCTGGGTGGCCGATC
CAAATCACACCACGCCCGCACTGGCAGAAATTCAGTATTGCTACGT
GAACGGTCGCATGATGCGCGATCGCCTGATCAATCACGCGATCCG
CCAGGCCTGCGAAGACAAACTGGGGGCCGATCAGCAACCGGCATT
TGTGTTGTATCTGGAGATCGACCCACATCAGGTGGACGTCAACGTG
CACCCCGCCAAACACGAAGTGCGTTTCCATCAGTCGCGTCTGGTGC
ATGATTTTATCTATCAGGGCGTGCTGAGCGTGCTACAACAGCAACT
GGAAACGCCGCTACCGCTGGACGATGAACCCCAACCTGCACCGCG
TTCCATTCCGGAAAACCGCGTGGCGGCGGGGCGCAATCACTTTGC
AGAACCGGCAGCTCGTGAGCCGGTAGCTCCGCGCTACACTCCTGC
GCCAGCATCAGGCAGTCGTCCGGCTGCCCCCTGGCCGAATGCGCA
GCCAGGCTACCAGAAACAGCAAGGTGAAGTGTATCGCCAGCTTTT
GCAAACGCCCGCGCCGATGCAAAAATTAAAAGCGCCGGAACCGCA
GGAACCTGCACTTGCGGCGAACAGTCAGAGTTTTGGTCGGGTACT
GACTATCGTCCATTCCGACTGTGCGTTGCTGGAGCGCGACGGCAAC
ATTTCACTTTTATCCTTGCCAGTGGCAGAACGTTGGCTGCGTCAGG
CACAATTGACGCCGGGTGAAGCGCCCGTTTGCGCCCAGCCGCTGCT
GATTCCGTTGCGGCTAAAAGTTTCTGCCGAAGAAAAATCGGCATTA
GAAAAAGCGCAGTCTGCCCTGGCGGAATTGGGTATTGATTTCCAGT
CAGATGCACAGCATGTGACCATCAGGGCAGTGCCTTTACCCTTACG
CCAACAAAATTTACAAATCTTGATTCCTGAACTGATAGGCTACCTG
GCGAAGCAGTCCGTATTCGAACCTGGCAATATTGCGCAGTGGATT
GCACGAAATCTGATGAGCGAACATGCGCAGTGGTCAATGGCACAG
GCCATAACCCTGCTGGCGGACGTGGAACGGTTATGTCCGCAACTTG
TGAAAACGCCGCCGGGTGGTCTGTTACAATCTGTTGATTTACATCC
GGCGATAAAAGCCCTGAAAGATGAGTGA1848 (SEQ ID NO: 4)
*E. coli* ruvA gene sequence 
TCATAACGCGGCGCGTAGGGCTTCGCGAATTAAAGTTTCACTGCTG
GCGTCAGGGCGAGCGATTTTGCTCACCATGCGGCTTGCTTCTTGTG
GTTTATAGCCCAGCGCCACCAGCGCGGCAACCGCTTCTTGTTCAGC
ATCGTCGGTCGCCGGGCTGGCAGGAGACGTGAGTACCAGGTCGGC
GGCTGGCGTAAAGAGATCGCCATGCAAACCTTTAAATCGGTCTTTC
ATTTCAACAATCAAGCGTTCGGCGGTTTTTTTGCCAATACCCGGCA
GTTTCACCAGTGCCCCCACTTCTTCACGCTCAACGGCATTAACGAA
CTGCTGCGCTGACATTCCGGAGAGGATCGCCAGCGCCAACTTCGG
GCCGACGCCGTTGGTTTTGATCAACTCTTTGAACAATGTGCGCTCT
TGTTTATTGTTAAAACCGTACAGCAGTTGCGCGTCTTCACGCACCA
CAAAGTGGGTGAAAACGATCGCTTCCTGACCCGCTTCAGGGAGTT
CATAAAAACAGGTCATCGGCATATGCACTTCATAGCCTACGCCGCC
CACTTCAATTAACACCAGCGGGGGTTGTTTTTCAATGATGATGCCT
CTGAGTCTGCCTATCAC612 (SEQ ID NO: 5)
*E. coli* ruvB gene sequence 
TTACGGCATTTCTGGCGGCGTTATGCCAAAGTGATTCCACGCCCGC
GTCGTCGCCATACGCCCACGCGGTGTACGCTGCAAAAAGCCTTGCT
GAATCAAATAAGGTTCCAGCACATCCTCAATGGTTTCACGTTCTTC
GCCAATGGCTGCCGCCAGGTTATCCAGACCTACAGGTCCACCAAA
GAACTTATCGATTACCGCCAGCAACAATTTGCGGTCCATATAATCG
AAACCTTCAGCATCGACATTCAACATATCCAGCGCCTGAGCAGCG
ATATCTGCCGAGATGGTGCCATCGTGCTTCACTTCGGCGAAATCAC
GCACTCGACGCAGCAGACGGTTGGCAATGCGCGGCGTACCGCGAG
CGCGACGAGCAACTTCCAGCGCGCCGTCATCACTCATCTCAAGCCC
CATAAAGCGTGCGCTGCGACTGACGATATATTGCAGATCCGGCAC
CTGATAAAACTCCAGACGTTGCACAATACCAAAACGGTCGCGCAA
CGGTGATGTCAGCGAACCTGCGCGCGTGGTTGCACCAATCAGGGT
AAACGGCGGCAAATCAATTTTAATGGAGCGTGCCGCCGGACCTTC
ACCAATCATGATATCCAGTTGGTAGTCTTCCATTGCCGGGTACAGC
ACTTCTTCAACAACTGGCGATAGACGGTGGATCTCATCAATAAACA
GCACGTCATGCGGTTCAAGGTTAGTGAGCATCGCAGCCAAATCGC
CCGCCTTTTCCAGCACCGGACCAGAAGTCGTGCGTAAATTAACGCC
CATTTCATTGGCGACAATGTTGGCAAGCGTAGTTTTACCCAACCCC
 
GGAGGACCAAAAATCAACAAATGATCGAGGGCATCGCCGCGCAGT
TTCGCTGCTTTGATGAAAATCTCCATCTGTGAACGAACCTGCGGCT
GACCAACATACTCTTCCAGTAATTTGGGGCGAATGGCGCGATCTGC
TACATCTTCCGGCAAAGTGGTACCGGCAGAAATCAGACGGTCTGC
TTCAATCAT1011 (SEQ ID NO: 6)
*E. coli* ruvC gene sequence 
TTAACGCAGTCGCCCTCTCGCCAGGTTCAGCCGCGATTCGCTCATC
TGCATCGCATTCTGACTAACGTGGCAGTGGGTGATAGCAATCGCCA
GCGCATCGGCGGCATCCGCCTGTGGATTAGCGGGCAGTTTCAGCA
AGGTGCGGACCATATGCTGCACCTGGCTTTTTTCGGCACTGCCAAT
ACCTACCACCGTTTGCTTTACCTGACGTGCCGCGTATTCAAATACT
GGCAACTCCTGATTCACCGCCGCCACAATCGCCACGCCGCGCGCCT
GGCCCAGTTTCAGGGCTGAGTCAGCGTTCTTTGCCATAAAGACTTG
TTCAATGGCGAAATAATCAGGCTGGAACTGGGTGATGATTTCCGTC
ACGCCCGCATAGATGAGTTTCAGACGAGACGGTAAATCATCCACT
TTGGTGCGGATGCATCCGCTACCCAGGTAGGACAGTTGCCTACCTA
CCTGGCGGATGACGCCGTAGCCGGTCACGCGCGAACCCGGATCAA
TGCCGAGAATAATAGCCAT522 (SEQ ID NO: 7)
*E. coli* lacIq gene sequence 
TCACTGCCCGCTTTCCAGTCGGGAAACCTGTCGTGCCAGCTGCATT
AATGAATCGGCCAACGCGCGGGGAGAGGCGGTTTGCGTATTGGGC
GCCAGGGTGGTTTTTCTTTTCACCAGTGAGACGGGCAACAGCTGAT
TGCCCTTCACCGCCTGGCCCTGAGAGAGTTGCAGCAAGCGGTCCAC
GCTGGTTTGCCCCAGCAGGCGAAAATCCTGTTTGATGGTGGTTAAC
GGCGGGATATAACATGAGCTGTCTTCGGTATCGTCGTATCCCACTA
CCGAGATATCCGCACCAACGCGCAGCCCGGACTCGGTAATGGCGC
GCATTGCGCCCAGCGCCATCTGATCGTTGGCAACCAGCATCGCAGT
GGGAACGATGCCCTCATTCAGCATTTGCATGGTTTGTTGAAAACCG
GACATGGCACTCCAGTCGCCTTCCCGTTCCGCTATCGGCTGAATTT
GATTGCGAGTGAGATATTTATGCCAGCCAGCCAGACGCAGACGCG
CCGAGACAGAACTTAATGGGCCCGCTAACAGCGCGATTTGCTGGT
GACCCAATGCGACCAGATGCTCCACGCCCAGTCGCGTACCGTCTTC
ATGGGAGAAAATAATACTGTTGATGGGTGTCTGGTCAGAGACATC
AAGAAATAACGCCGGAACATTAGTGCAGGCAGCTTCCACAGCAAT
 
GGCATCCTGGTCATCCAGCGGATAGTTAATGATCAGCCCACTGACG
CGTTGCGCGAGAAGATTGTGCACCGCCGCTTTACAGGCTTCGACGC
CGCTTCGTTCTACCATCGACACCACCACGCTGGCACCCAGTTGATC
GGCGCGAGATTTAATCGCCGCGACAATTTGCGACGGCGCGTGCAG
GGCCAGACTGGAGGTGGCAACGCCAATCAGCAACGACTGTTTGCC
CGCCAGTTGTTGTGCCACGCGGTTGGGAATGTAATTCAGCTCCGCC
ATCGCCGCTTCCACTTTTTCCCGCGTTTTCGCAGAAACGTGGCTGG
CCTGGTTCACCACGCGGGAAACGGTCTGATAAGAGACACCGGCATA
CTCTGCGACATCGTATAACGTTACTGGTTTCAC1083 (SEQ ID NO: 8)
*E. coli* lacIq1 gene: 
ACCTTTCGCGGTATGGCATGATAGCGCCCGGAAGAGAGTCAATTC
AGGGTGGTGAATGTGAAACCAGTAACGTTATACGATGTCGCAGAG
TATGCCGGTGTCTCTTATCAGACCGTTTCCCGCGTGGTGAACCAGG
CCAGCCACGTTTCTGCGAAAACGCGGGAAAAAGTGGAAGCGGCGA
TGGCGGAGCTGAATTACATTCCCAACCGCGTGGCACAACAACTGG
CGGGCAAACAGTCGTTGCTGATTGGCGTTGCCACCTCCAGTCTGGC
CCTGCACGCGCCGTCGCAAATTGTCGCGGCGATTAAATCTCGCGCC
GATCAACTGGGTGCCAGCGTGGTGGTGTCGATGGTAGAACGAAGC
GGCGTCGAAGCCTGTAAAGCGGCGGTGCACAATCTTCTCGCGCAA
CGCGTCAGTGGGCTGATCATTAACTATCCGCTGGATGACCAGGATG
CCATTGCTGTGGAAGCTGCCTGCACTAATGTTCCGGCGTTATTTCTT
GATGTCTCTGACCAGACACCCATCAACAGTATTATTTTCTCCCATG
AAGACGGTACGCGACTGGGCGTGGAGCATCTGGTCGCATTGGGTC
ACCAGCAAATCGCGCTGTTAGCGGGCCCATTAAGTTCTGTCTCGGC
GCGTCTGCGTCTGGCTGGCTGGCATAAATATCTCACTCGCAATCAA
ATTCAGCCGATAGCGGAACGGGAAGGCGACTGGAGTGCCATGTCC
GGTTTTCAACAAACCATGCAAATGCTGAATGAGGGCATCGTTCCCA
CTGCGATGCTGGTTGCCAACGATCAGATGGCGCTGGGCGCAATGC
GCGCCATTACCGAGTCCGGGCTGCGCGTTGGTGCGATATCTCGGTA
GTGGGATACGACGATACCGAAGACAGCTCATGTTATATCCCGCCG
TTAACCACCATCAAACAGGATTTTCGCCTGCTGGGGCAAACCAGC
GTGGACCGCTTGCTGCAACTCTCTCAGGGCCAGGCGGTGAAGGGC
AATCAGCTGTTGCCCGTCTCACTGGTGAAAAGAAAAACCACGCTA
GCGCCCAATACGCAAACCGCCTCTCCCCGCGCGTTGGCCGATTCAT
 
TAATGCAGCTGGCACGACAGGTTTCCCGACTGGAAAGCGGGCAGT
GA1139 (SEQ ID NO: 9)
Adeno-associated virus 2 ITR sequence aggaacccctagtgatggagttggccactccctctctgcgcgctcgctcgctcactgaggccgcccgggcaa agcccgggcgtcgggcgacctttggtcgcccggcctcagtgagcgagcgagcgcgcagagagggagtgg ccaa (SEQ ID NO: 10)
Targeting sequence of sgRNA for sbcC gene
   ttccgcgcgttctttgggtt 20 (SEQ ID NO: 11)
Targeting sequence of sgRNA for sbcD gene
   cgattgaacacctcttcgac 20 (SEQ ID NO: 12)
Targeting sequence of sgRNA for nfi gene
   atctcgcatcctcgccgtct 20 (SEQ ID NO: 13)
Targeting sequence of sgRNA for mutL gene
   attatcagcctgggctttcg 20 (SEQ ID NO: 14)
Targeting sequence of sgRNA for ruvA gene
   ttcaacaatcaagcgttcgg 20 (SEQ ID NO: 15)
Targeting sequence of sgRNA for ruvB gene
   cggaggaccaaaaatcaaca 20 (SEQ ID NO: 16)
Targeting sequence of sgRNA for ruvC gene
   tgcggaccatatgctgcacc 20 (SEQ ID NO: 17)
Targeting sequence of sgRNA for lacIq gene
   gatgaacgtgccggaaagcg 20 (SEQ ID NO: 18)
Targeting sequence of sgRNA for lacIq1 gene
   gccggataagcctcgctttc 20 (SEQ ID NO: 19)
ITR sequence of pITR-test1 and pITR-test3 
ttggccactccctctctgcgcgctcgctcgctcactgaggccgggcgaccaaaggtcgcccgacgcccgggc
tttgcccgggcggcctcagtgagcgagcgagcgcgcagagagggagtggccaa125 (SEQ ID NO:
20)
ITR sequence of pITR-test2 
gccactccctctctgcgcgctcgctcgctcactgaggccgggcgaccaaaggtcgcccgacgcccgggcttt
gcccgggcggcctcagtgagcgagcgagcgcgcagagagggagtggc119 (SEQ ID NO: 21)
Donor sequence of sbcC gene 
CACGATCGCTCCGACGCCAAAAAATGCGCCATGCGGAAAGCCGGA
TACCAGCCGACCAATGGCGAGCATCAGGTAAGACGAAGAGAGCGT
 
GAACATGGCGTTGCCAATGACGCACAACGCCACCAGAAACAACAA
GATATGTTTGAGTGAGTAGCGGCTGGAAAAGAGTGCGATGATTGG
CGCACCGACCACCACCCCCAGTGCATAATACGAGATCATATGCCC
GGCGGCAGGAATCGAAATTCCTACGTTATGCGCCAGCTCCGTGAG
CACGCCCATAATGCCAAATTCGGCCATCCCCAAACCAAACGTGCC
CAGAGCCAAAGATAAAATGACTTTTTTCATACCACCATCACAACCA
CCAGAACGGCTACCAATCACCGTAGCCAATAAAGTGATAGCTTGC
AGGATAACGACCAGCAAGGCCAAGAGATAATTCGCCCCTCTGTAT
TCATTATCCTGCTGAATAGTTATTTCACTGCAAACGTACTTTCCAGT
TTGCTCCCGTTGCTGGCGAACGGCTCGCGGGTGAAATCAATCTTCC
ATTCGCCTTTTAATGAGTTCAGGTTTTTCAGGCGCAGGCTGAGAAT
TTTCATGCTTCGTGTTCTCCGGCGAGGGTATGCAACGTCGTGGTGA
AAAGATGCTGCAGACGTTGCTGCTGCGATTCATCCAGTTCTTCCAG
TGCCAGACGGCGATTGAACACCTCTTCGACGCTGAGTTCGCTGAGG
GTTTCACGCTGTTGGCTGGCTAACACACGCTCGCGCTGTTCACGAC
TCCGACGTACCAGCAATACTTCGACAGGCAATGATTCGGTTAATGC
CTGGATTTTGCGCTGAATATCATGCAGATACTCATCAGTAGTGATT
TCGATATCCAGCCAGACAGGTGGCTCCTGCGATACATCGCGCCACT
GTTCCAGCTGTGCGGTAATCGACGCCAGATCGCCTTTCAGCACTGC
CATGGGTTGCGTTACCGGTACGTTCAGGTTTTCCACGCTCTCTAA10
03 (SEQ ID NO: 22)
Donor sequence of sbcD gene 
CGTCGCTGTCAGTTCCAGCTTATCTTTCACTTTGTCGGCGAGAATTT
TGCCGTCGGCGCAGCGCGCCAGCTCTACGCGTGGCACCTGCAAATT
ACCGTCGGGTTGGTTACGCGCCCGATTCTGGCTCCAGAATGCACGG
TACGCTTCACCTTTCACTTCAAACTCCACCTCCGCCAGACATTCGG
CGGTATCGCGGGTCATGAGATCATTTTGCGATTGTGAAACGTTAGA
GAGACGCGGAGTTTCGTGATACAGCGCCAGACAAATGGCGTCCAG
CAGGGTGGTTTTCCCCGCACCTGTTGGGCCGGTAATAGCAAACAGC
CCGTTGCTGGCGAACGGCTCGCGGGTGAAATCAATCTTCCATTCGC
CTTTTAATGAGTTCAGGTTTTTCAGGCGCAGGCTGAGAATTTTCAT
GCTTCGTGTTCTCCGGCGAGGGTATGCAACGTCGTGGTGAAAAGAT
GCTGCAGACGTTGCTGCTGCGATTCATCCAGTTCTTCCAGCTGGCC
GAGATGCCAGTCTGAGGTGTGAAGGATGCGCATAACGGTTCCCTG
 
GCGAAAAAGCATGGGCGCGATTATACCCAAACAGATGTGCCATTT
GCTTTTTTCTGCGCCACGGAAATCAATAACCTGAAGATATGTGCGA
CGAGCTTTTCATAAATCTGTCATAAATCTGACGCATAATGACGTCG
CATTAATGATCGCAACCTATTTATTACAACAGGGCAAATCATGGCG
AGACGTATTCTGGTCGTAGAAGATGAAGCTCCAATTCGCGAAATG
GTCTGCTTCGTGCTCGAACAAAATGGCTTTCAGCCGGTCGAAGCGG
AAGATTATGACAGTGCTGTGAATCAACTGAATGAACCCTGGCCGG
ATTTAATTCTCCTCGACTGGATGTTACCTGGCGGCTCCGGTATCCA
GTTCATCAAACACCTCAAGCGCGAGTCGATGACCCGGGATATTCC
AGTGGTGATGTTGACCGCCAGAGGGGAAGAAGATCG1000
(SEQ ID NO: 23)
Donor sequence of nfi gene 
AACAGTTCTCGCTCTATTACATGCATAAAATTGTTGATGGTTTACT
GCGTGAAAACGACGGTCGCCGCGTACCGGTCACGCTGTTTACCAA
AGGCGGCGGACAGTGGCTGGAAGCCATGGCAGAAACCGGTTGCGA
TGCGCTGGGCCTCGACTGGACAACGGACATCGCCGATGCGCGCCG
CCGTGTGGGCAATAAAGTCGCGTTGCAGGGTAATATGGATCCGTC
GATGCTGTACGCGCCGCCTGCCCGCATTGAAGAAGAAGTAGCGAC
TATACTTGCAGGTTTCGGTCACGGCGAAGGTCATGTCTTTAACCTT
GGTCACGGCATTCATCAGGATGTGCCGCCAGAACATGCTGGCGTG
TTCGTGGAGGCAGTGCATCGACTGTCTGAACAGTATCACCGCTAAG
GAGTGATTATGGATCTCGCGTCATTACGCGCTCAACAAATCGAACT
GGCTTCTTCTGTGATCCGCGAGGATCGACTCGATAAAGATCCACCG
CGTCTGCCGGAGCCAACGCGCTGGGCGGACGCGGTGGCCTCGGAA
CGTCCGGCGTTCGTGCGCTATACAGCAAATCAGCCCTAATTCAGGT
AAACTGCGGATAATTTCCGTATTTGAGAACTCAACATGTTACAAAA
CCCAATTCATCTGCGTCTGGAGCGCCTGGAAAGCTGGCAGCATGTC
ACTTTCATGGCTTGCTTATGCGAACGCATGTACCCCAATTACGCCA
TGTTCTGCCAGCAAACCGGTTTTGGTGATGGGCAAATTTACCGTCG
CATTCTCGATCTCATCTGGGAAACGCTGACCGTTAAAGACGCAAA
AGTAAATTTCGACAGCCAACTGGAGAAATTTGAAGAAGCGATTCC
TTCAGCCGACGATTTCGATCTGTACGGCGTTTATCCGGCAATCGAT
GCCTGCGTGGCGTTAAGTGAACTGGTCCATTCGCGTTTGAGTGGTG
 
AAACGCTCGAACACGCGGTGGAAGTGAGTAAGACCTCCATCAC100
0 (SEQ ID NO: 24)
Donor sequence of mutL gene 
TGCTGGGTGGGGCGGGTGATGTGCTGGCGAACAGTCAGTCTGACC
CCTATTTAAGCCAGGCGGTGCTGGATTTACAGTTCGGTCATTCCCA
GCGGGTAGGGTATGATGTAGCGACCAGTATGATCAGTCAGTTGCA
ACGCATTGGCGAAATACATAAACGTCGACCAGAACACGCCAGCCT
TGGCGTTCTGCGCTCGCCGGATATCCCATCAGTACTGGTCGAAACC
GGTTTTATCAGCAACAACAGCGAAGAACGTTTGCTGGCGAGCGAC
GATTACCAACAACAGCTGGCAGAAGCCATTTACAAAGGCCTGCGC
AATTACTTCCTTGCGCATCCGATGCAATCTGCGCCGCAGGGTGCAA
CGGCACAAACTGCCAGTACGGTGACGACGCCAGATCGCACGCTGC
CAAACTAAGGACGATTGATGCCAATTCAGGTCTTACCGCCACAACT
GGCGAACCAGATTGCCGCAGGTGAGGTGGTCGAGCGACCTGCGTC
GCTTGTGAAAACGCCGCCGGGTGGTCTGTTACAATCTGTTGATTTA
CATCCGGCGATAAAAGCCCTGAAAGATGAGTGATATCAGTAAGGC
GAGCCTGCCTAAGGCGATTTTTTTGATGGGGCCGACGGCCTCCGGT
AAAACGGCGTTAGCCATTGAGCTGCGTAAAATTTTACCAGTAGAG
TTGATAAGCGTTGATTCTGCCCTTATTTACAAAGGGATGGATATCG
GGACGGCGAAGCCGAACGCTGAAGAGTTACTCGCCGCGCCGCACC
GATTGCTGGATATTCGCGATCCGTCGCAGGCTTACTCGGCTGCTGA
TTTTCGCCGCGATGCGCTGGCGGAAATGGCCGATATCACCGCGGC
GGGGCGGATCCCACTGTTAGTGGGCGGTACGATGTTGTATTTCAAG
GCATTGCTGGAAGGGTTGTCGCCGCTACCGTCGGCAGACCCGGAA
GTACGGGCCAGAATTGAGCAACAGGCGGCAGAGCAAGGTTGGGA
GT1000 (SEQ ID NO: 25)
Donor sequence of ruvA gene 
CGGCAAATCAATTTTAATGGAGCGTGCCGCCGGACCTTCACCAATC
ATGATATCCAGTTGGTAGTCTTCCATTGCCGGGTACAGCACTTCTT
CAACAACTGGCGATAGACGGTGGATCTCATCAATAAACAGCACGT
CATGCGGTTCAAGGTTAGTGAGCATCGCAGCCAAATCGCCCGCCTT
TTCCAGCACCGGACCAGAAGTCGTGCGTAAATTAACGCCCATTTCA
TTGGCGACAATGTTGGCAAGCGTAGTTTTACCCAACCCCGGAGGA
CCAAAAATCAACAAATGATCGAGGGCATCGCCGCGCAGTTTCGCT
 
GCTTTGATGAAAATCTCCATCTGTGAACGAACCTGCGGCTGACCAA
CATACTCTTCCAGTAATTTGGGGCGAATGGCGCATCTGCTACATC
TTCCGGCAAAGTGGTACCGGCAGAAATCAGACGGTCTGCTTCAAT
CATCCTTTACCTCATAACGCGGCGCGTAGGGCTTCGCGAATTAACA
TATGCACTTCATAGCCTACGCCGCCCACTTCAATTAACACCAGCGG
GGGTTGTTTTTCAATGATGATGCCTCTGAGTCTGCCTATCACATGA
CGCTCCTGCGTAATGAATCAAAGATAATGCTGTATGATAAAAAAA
TGCTGGATAGATATCCAGCGAAGGATGAAGAAAACTTGCGAGGTG
TCTCGATGAACTGAAAAATGGCACAGTATAATTTATTCTACAGGTT
ATATTGGAAGCAAATATTTTAATATTACATATTCAGTGAAGAAATG
CGTAATAAAAATATACATTGCGCCTCCTGAAAAAATAAATTTTTTA
TGCTATTACGTATATTTGTATCTATTTCAATGGAATGACAACGTGA
ATATTAATTACCCTGCTGAATATGAAATTGGAGATATCGTCTTTAC
ATGTATAGGTGCTGCCTTATTTGGTCAAATATCAGCTGCATCAAAT
TGCTGGAGTAATCACGTCGGGATCATTATCGGTCATAACG1000
(SEQ ID NO: 26)
Donor sequence of ruvB gene 
CGAGTGAAATTGTTGTTGATGCTGGTGACGGCATTGACGATTGGTG
TAGCGATGAAATTTGTCGGTGCGTTGATTATTACTTCGTTGCTGATT
ATTCCTGCTGCTACTGCGCGTCGCTTTGCCCGCACGCCGGAACAGA
TGGCTGGTGTCGCTGTTTTGGTGGGGATGGTGGCAGTGACTGGCGG
TTTAACCTTTTCCGCGGTTTACGATACGCCGGCGGGTCCGTCGGTG
GTGCTATGTGCGGCACTGTTATTTATTCTCAGTATGATGAAAAAGC
AGGCCAGCTAATCTATCGCTGAACACATTTGTCGGATGCGGCGCG
AGCGCCTTATCCGACCTACGGTTCGGTATCTCTGGTAGTCCTGGTA
AGACGCGAACAGCGTCGCATCAGGCATATTGCCAGTGCCGGATGC
GGCGCGAGCGACCAATCCGACTTACGGCATTTCTGGCGGCGTTATG
CCAAAGTGATTCCACGCCCGCGTCGTCGCCATACGCCCACGGCGA
ATGGCGCGATCTGCTACATCTTCCGGCAAAGTGGTACCGGCAGAA
ATCAGACGGTCTGCTTCAATCATCCTTTACCTCATAACGCGGCGCG
TAGGGCTTCGCGAATTAAAGTTTCACTGCTGGCGTCAGGGCGAGC
GATTTTGCTCACCATGCGGCTTGCTTCTTGTGGTTTATAGCCCAGCG
CCACCAGCGCGGCAACCGCTTCTTGTTCAGCATCGTCGGTCGCCGG
GCTGGCAGGAGACGTGAGTACCAGGTCGGCGGCTGGCGTAAAGAG
 
ATCGCCATGCAAACCTTTAAATCGGTCTTTCATTTCAACAATCAAG
CGTTCGGCGGTTTTTTTGCCAATACCCGGCAGTTTCACCAGTGCCC
CCACTTCTTCACGCTCAACGGCATTAACGAACTGCTGCGCTGACAT
TCCGGAGAGGATCGCCAGCGCCAACTTCGGGCCGACGCCGTTGGT
TTTGATCAACTCTTTGAACAATGTGCGCTCTTGTTTATT1000 (SEQ
ID NO: 27)
Donor sequence of ruvC gene 
AAGCCGGGTTCCCCTTTCAACCATCACCACGCTATCTCGTTTTATTA
AACGTTCGTCTAATCAACGCTATGCTATAAAGCGATTAGACGCTGG
GCTGACAGAACGACAAAAACAACGAATTGTTGAACAGGTTCCCTC
CCGGCTACGCAAACTTTACCACACCGGTTTTAAATACGAATCTTCG
CGCCAGTTCTGTTCAAAATTTGTTTTTGATATTTATAAAGAAGCGC
TATGTATTCCGGTGGGTGAAATAGAGACGTTTGGAGAATTGTTAAA
TAGCAATCCGAATGCAAAACTCACTTTCTGGAAATTCTGGTTCCTG
GGTTCTATTCCGTGGGAGCGTAAAACCGTCACGCCAGCCAGTTTGT
GGCATCATCCGGGTTTGGTGTTGATTCATGCGGAGGGAGTGGAAA
CGCCTCAGCCGGAACTGACCGAGGCGGTATAACTTAACGCAGTCG
CCCTCTCGCCAGGTTCAGCCGCGATTCGCTCATCTGCATCGCGGTC
ACGCGCGAACCCGGATCAATGCCGAGAATAATAGCCATCACGCGT
CTCCGTTTTGCTGTTTAGCAGGCCTCATCAGAGAGTCGCTGCGACC
TCATCAGAGATTTCACCGTTATGGTAAACTTCCTGCACGTCGTCGC
AATCTTCCAGCATATCGATCAGACGCATCAGTTTCGGTGCGGTTTC
TGCATCCATATCAGCTTTGGTAGACGGGATCATGGAAACTTCCGCG
CTGTCTGCTTTCAGACCTGCCGCTTCCAGAGCGTCGCGCACTTTAC
CCATTTCTTCCCATGCAGTGTAGACATCAATCGCGCCGTCATCATA
GGTCACAACGTCTTCAGCACCTGCTTCCAGTGCTGCTTCCATGATG
GTGTCTTCATCGCCTTTCTCGAAGGAGATCACGCCTTTTTTGCTGAA
CAGATAGGCAACGGAACCATCAGTACCGAGGTTACCGCCACATTT
GCTAAATGCATGACGCACTTCAGCAACGGTACGGTTG1000 (SEQ ID
NO: 28)
Donor sequence of lacIq gene 
TGGTTATCGATATCATGATACTGCGGCGGGGCGCTAAAACGAAGG
GAAGAAGCATTAGTGTAATTAATTAAAGCAAGATAATACGTATGT
TTGATCAATTTTCGAGGCGATAACCGGCCATAAAAAAGCGCAACG
 
TAGAACAGGAATTATTCTCACGAAAGAAATCCATTACCATCTCTTT
ATCCAGTCCATAGCGGCGCGTCAGAATACCCGCTTCCCAGGCGCTA
AGCTGGCGATCTCCGGTCTTTTCGTACATCCGGTGTGAGTAGCCTA
ATACAAAACCGCGTTTATAATCGGCACAGAATTTTGTCACATTAAC
CGCACTGTCGGCATGCGTTGCTTTTAATCCCGCCATTAAGCCTTTA
CCAAAATGGTTATTCATCGTTCACCTCATTCGCTATATATTGTCATA
TATAGCGATATTTTAGGCAAAGAACGACTTTTGCGCACGCTATTTT
TTCATGCTTGTTTAAGACGTAATCCCGTAAGAGATAAACCTATTCC
GACACCATCGAATGGTGCAAAACCTTTCGCGGTATGGCATGATAG
CGCCCGGAAGAGAGTCAATTCAGGGTGGTGAATGTGAAACCAGTA
ACGTTATACGATGTCGCAGAGTATGCCGGTGTCTCTTATCAGACCG
TTTCCCGCGTGGTGAACCAGGCCAGCCACGTTTCTGCGAAAACGCG
GGAAAAAGTGGAAGCGGCGATGGCGGAGCTGAATTACATTCCCAA
CCGCGTGGCACAACAACTGGCGGGCAAACAGTCGTTGCTGATTGG
CGTTGCCACCTCCAGTCTGGCCCTGCACGCGCCGTCGCAAATTGTC
GCGGCGATTAAATCTCGCGCCGATCAACTGGGTGCCAGCGTGGTG
GTGTCGATGGTAGAACGAAGCGGCGTCGAAGCCTGTAAAGCGGCG
GTGCACAATCTTCTCGCGCAACGCGTCAGTGGGCTGATCATTAACT
ATCCGCTGGATGACCAGGATGCCATTGCTGTGGAAGCTGCCTGCAC
TAATGTTCCGGCGTTATTTCTTGATGTCTCTGACCAGACACCCATC
AACAGTATTATTTTCTCCCATGAAGACGGTACGCGACTGGGCGTGG
AGCATCTGGTCGCATTGGGTCACCAGCAAATCGCGCTGTTAGCGG
GCCCATTAAGTTCTGTCTCGGCGCGTCTGCGTCTGGCTGGCTGGCA
TAAATATCTCACTCGCAATCAAATTCAGCCGATAGCGGAACGGGA
AGGCGACTGGAGTGCCATGTCCGGTTTTCAACAAACCATGCAAAT
GCTGAATGAGGGCATCGTTCCCACTGCGATGCTGGTTGCCAACGAT
CAGATGGCGCTGGGCGCAATGCGCGCCATTACCGAGTCCGGGCTG
CGCGTTGGTGCGGATATCTCGGTAGTGGGATACGACGATACCGAA
GACAGCTCATGTTATATCCCGCCGTTAACCACCATCAAACAGGATT
TTCGCCTGCTGGGGCAAACCAGCGTGGACCGCTTGCTGCAACTCTC
TCAGGGCCAGGCGGTGAAGGGCAATCAGCTGTTGCCCGTCTCACT
GGTGAAAAGAAAAACCACCCTGGCGCCCAATACGCAAACCGCCTC
TCCCCGCGCGTTGGCCGATTCATTAATGCAGCTGGCACGACAGGTT
TCCCGACTGGAAAGCGGGCAGTGAAATTCGCGCAACGCAATTAAT
 
GTAAGTTAGCTCACTCATTAGGCACCAAATAAAACGAAAGGCTCA
GTCGAAAGACTGGGCCTTTCGTTTTATCTGTTGTTTGTCGGTGAAC
GCTCTCCTGAGTAGGACAAATCCGCCGGGAGCGGATTTGAACGTT
GCGAAGCAACGGCCCGGAGGGTGGCGGGCAGGACGCCCGCCATA
AACTGCCAGGCATCAAATTAAGCAGAAGGCCATCCTGACGGATGG
CCTTTTAGTAATCCAGCAACTCTTGTGGGAAATCTTTGGCGGTTAA
ACGCGGCATCGTTGCACTCCTCAGTTGGTGTTTTTTTAAGCAAAGC
ATAAGCACGTATTTTTGCCCAGTTTTTCGTCACTCTGTGAGCCAGA
CTACGGGATACGCGCTGGCGAATCGCTAAACTAGAAACATTGTTTC
GAAATTGAACGGTGGAAAGGAGAGGTCATGGTCTGGTTAGCGAAT
CCCGAACGTTACGGGCAGATGCAATACCGCTATTGCGGAAAAAGT
GGTTTACGCCTGCCCGCGTTATCGCTCGGTTTATGGCACAATTTCG
GTCACGTTAACGCGCTGGAATCACAGCGTGCGATCCTGCGTAAAG
CGTTTGATTTGGGCATTACGCACTTTGATTTAGCCAACAATTACGG
GCCGCCTCCAGGAAGCGCAGAAGAGAACTTTGGTCGCCTGCTGCG
GGAGGATTTTGCCGCTTATCGCGATGAACTGATTATCTCTACCAAG
GCTG2417 (SEQ ID NO: 29)
Donor sequence of lacIq1 gene

CAAACCTGGGGGTTGTTCTTCGAACGCCTGAATGTATCGGTACAGA
AACTGGAAAACGCCATCGAGTTTTATGGCGCGATTTCGCATAACA
AACTGCACGGCCTGTCAGTGCAGGTTGAAACCAATCACGTCAAAT
TACCGGCGGTGGTGGACGGCTTTGCCAGCGAAGGGATCATCCAGT
TCTTTTTCGAAGAAACGCAAGACGAGAATGGCTTTAATATCTACAT
TCTCGACGAAAGCAACCGGGTTGAGGTATATCACCACTGCGAAGG
CAGCAAAGAGGAGCTGGTACGTGACGTCAGTCGCTTCTACTCGTC
ATCGCATGACCGcTTTACCTACGGCTCAAGCTTCATCAACTTCAAC
CTGCCGCAGTTCTATCAGATTGTGAAGGTTGATGGTCGTGAACAGG
TGATTCCGTTCCGCACAAAATCTATCGGTAACATGCCGCCTGCCAA
TCAGGATCACGATACGCCGCTATTACAGCAATATTTTTCGTGATGA
ACGTGGACACCATCGAATGGTGCAAAACCTTTCGCGGTATGGCAT
GATAGCGCCCGGAAGAGAGTCAATTCAGGGTGGTGAATGTGAAAC
CAGTAACGTTATACGATGTCGCAGAGTATGCCGGTGTCTCTTATCA
GACCGTTTCCCGCGTGGTGAACCAGGCCAGCCACGTTTCTGCGAAA
ACGCGGGAAAAAGTGGAAGCGGCGATGGCGGAGCTGAATTACATT

CCCAACCGCGTGGCACAACAACTGGCGGGCAAACAGTCGTTGCTG
ATTGGCGTTGCCACCTCCAGTCTGGCCCTGCACGCGCCGTCGCAAA
TTGTCGCGGCGATTAAATCTCGCGCCGATCAACTGGGTGCCAGCGT
GGTGGTGTCGATGGTAGAACGAAGCGGCGTCGAAGCCTGTAAAGC
GGCGGTGCACAATCTTCTCGCGCAACGCGTCAGTGGGCTGATCATT
AACTATCCGCTGGATGACCAGGATGCCATTGCTGTGGAAGCTGCCT
GCACTAATGTTCCGGCGTTATTTCTTGATGTCTCTGACCAGACACC
CATCAACAGTATTATTTTCTCCCATGAAGACGGTACGCGACTGGGC
GTGGAGCATCTGGTCGCATTGGGTCACCAGCAAATCGCGCTGTTAG
CGGGCCCATTAAGTTCTGTCTCGGCGCGTCTGCGTCTGGCTGGCTG
GCATAAATATCTCACTCGCAATCAAATTCAGCCGATAGCGGAACG
GGAAGGCGACTGGAGTGCCATGTCCGGTTTTCAACAAACCATGCA
AATGCTGAATGAGGGCATCGTTCCCACTGCGATGCTGGTTGCCAAC
GATCAGATGGCGCTGGGCGCAATGCGCGCCATTACCGAGTCCGGG
CTGCGCGTTGGTGCGGATATCTCGGTAGTGGGATACGACGATACC
GAAGACAGCTCATGTTATATCCCGCCGTTAACCACCATCAAACAG
GATTTTCGCCTGCTGGGGCAAACCAGCGTGGACCGCTTGCTGCAAC
TCTCTCAGGGCCAGGCGGTGAAGGGCAATCAGCTGTTGCCCGTCTC
ACTGGTGAAAAGAAAAACCACGCTAGCGCCCAATACGCAAACCGC
CTCTCCCCGCGCGTTGGCCGATTCATTAATGCAGCTGGCACGACAG
GTTTCCCGACTGGAAAGCGGGCAGTGAGCGCAACGCAATTAATGT
AAGTTAGCTCACTCATTAGGCACCAAATAAAACGAAAGGCTCAGT
CGAAAGACTGGGCCTTTCGTTTTATCTGTTGTTTGTCGGTGAACGC
TCTCCTGAGTAGGACAAATCCGCCGGGAGCGGATTTGAACGTTGC
GAAGCAACGGCCCGGAGGGTGGCGGGCAGGACGCCCGCCATAAA
CTGCCAGGCATCAAATTAAGCAGAAGGCCATCCTGACGGATGGCC
TTTTATGCAATCTTAGCGGAAACTGACTGTTTCACCCGCCTGCTGC
GTCGCCGCCTGTTCCAGCAAATCCCAGAAGGTTTCGCCGCTGCGAT
CACAAATCCACTCATCGCCTTTCAGGTCAAAATGGTAGCCGCCCTG
TTTGGTTGCCAGCCATACCTGGTGCAGCGGCTCCTGGCGGTTGATA
ATGATTTTGCTGCCATTCTCAAAGGTAATGGTCAGTACGCCGCCGT
TGATTTCGCAGTCGATATCGCTGTCGCCATCCCAGTCGTCCAGGCG
TTCTTCAATGGTCAGCCAGAGTTGATCAGCCAGGCGATGAAATTCA
CTGTCGTTCATTGTTGTATCCTGTTTTTAAGTGATGGCGGCAGTATA

GCGGCATGGGGTCAGGGCTTCAAAGTTTGCACCTCTGCGGCTGCGT
TCCGGCACGATTCATCCGTCACCGGAATAATGATGTCTCTGTGTAG
CGAAAGATTTGTCTCTTCATTAGGGCGCAGTTACACCAC2410 (SEQ
ID NO: 30)

## Claims

1. A host cell, **characterized in that** the genome of the host cell has sbcC and/or sbcD genes knocked out, and the host cell further comprises the following modifications:
1) one or more of the nfi, mutS, mutL, mutH, ruvA, ruvB, and ruvC genes are knocked out in the genome of the host cell; and/or
2) the lacIq or lacIq1 gene is knocked into the genome of the host cell.

2. The host cell of claim 1, **characterized in that** in the genome of the host cell:
1) the sbcC and/or sbcD genes are knocked out, and the nfi gene is knocked out;
2) the sbcC and/or sbcD genes are knocked out, and at least one of the ruvA, ruvB, and ruvC genes is knocked out;
3) the sbcC and/or sbcD genes are knocked out, and at least one of the mutS, mutL, and mutH genes is knocked out; or
4) the sbcC and/or sbcD genes are knocked out, and the lacIq or lacIq1 gene is knocked in.

3. The host cell of claim 1 or 2, **characterized in that** in the genome of the host cell:
1) the sbcC and sbcD genes are knocked out, and the nfi gene is knocked out;
2) the sbcC and/or sbcD genes are knocked out, and the ruvA and ruvB genes are knocked out;
3) the sbcC and/or sbcD genes are knocked out, and the ruvC gene is knocked out;
4) the sbcC and/or sbcD genes are knocked out, and the ruvA, ruvB, and ruvC genes are knocked out;
5) the sbcC and/or sbcD genes are knocked out, and the mutL gene is knocked out;
6) the sbcC and/or sbcD genes are knocked out, and the mutL and nfi genes are knocked out; or
7) the sbcC and/or sbcD genes are knocked out, the mutL gene is knocked out, and the lacIq or lacIq1 gene is knocked in.

4. The host cell of any one of claims 1-3, **characterized in that** in the genome of the host cell:
1) the sbcC and sbcD genes are knocked out, and the ruvA, ruvB, and ruvC genes are knocked out; or
2) the sbcC and sbcD genes are knocked out, and the ruvC gene is knocked out.

5. The host cell of any one of claims 1-4, **characterized in that** the lacIq gene comprises the nucleotide sequence set forth in SEQ ID NO: 8, or a nucleotide sequence having at least 90% sequence identity to the nucleotide sequence set forth in SEQ ID NO: 8; and the lacIq1 gene comprises the nucleotide sequence set forth in SEQ ID NO: 9, or a nucleotide sequence having at least 90% sequence identity to the nucleotide sequence set forth in SEQ ID NO: 9.

6. The host cell of any one of claims 1-5, **characterized in that** the host cell is an *E. coli* cell.

7. The host cell of claim 6, **characterized in that** the *E. coli* cell is a Top10, JM108, or NEB Stable cell.

8. Use of the host cell of any one of claims 1-7 in the preparation of a nucleic acid molecule comprising a hairpin structure.

9. The use of claim 8, **characterized in that** the nucleic acid molecule is a plasmid.

10. The use of claim 8 or 9, **characterized in that** the hairpin structure is an ITR sequence, preferably an ITR derived from an adeno-associated virus.

11. The use of claim 10, **characterized in that** the number of nucleotides in the A region in the ITR sequence is at least 20; preferably at least 30; and more preferably at least 40.

12. The use of any one of claims 8-11, **characterized in that** the host cell is an *E. coli* cell; preferably a Top10, JM108, or NEB Stable cell.

13. The use of any one of claims 8-12, **characterized in that** the hairpin structure has stability in the host cell, including replication stability and/or passage stability.
